# EUROPÄISCHE PATENTSCHRIFT

(11) **EP 0 696 597 B1**
(45) Veröffentlichungstag und Bekanntmachung des Hinweises auf die Patenterteilung: **26.03.2008**
(21) Anmeldenummer: 95112393.4
(22) Anmeldetag: 07.08.1995
(51) Int. Cl.: C07K 16/18, C12N 5/20, G01N 33/53

(54) **Monoklonale Antikörper zur selektiven immunologischen Bestimmung von hochmolekularen, intakten Lamininformen in Körperflüssigkeiten**
Monoclonal antibodies for selective immunological determination of high molecular weight intact laminin forms in body fluids
Anticorps monoclonaux pour la détermination immunologique sélective des formes de la laminine intacte à haut poids moléculaire dans des fluides corporels

(30) Priorität: 11.08.1994 DE 4428481
(43) Veröffentlichungstag der Anmeldung: 14.02.1996
(73) Patentinhaber: Sanofi-Aventis Deutschland GmbH, 65929 Frankfurt am Main (DE)
(72) Erfinder: Gerl, Martin, Dr., D-65830 Kriftel (DE); Steinert, Cornelia, D-65207 Wiesbaden (DE); Quint, Manfred, Dr., D-65193 Wiesbaden (DE); Timpl, Rupert, Dr., D-82131 Gauting (DE)

(56) Entgegenhaltungen:
- DE-A- 3 331 627
- IWATA K: "ONE-STEP SANDWICH ENZYME IMMUNOASSAY FOR HUMAN LAMININ USING MONOCLONAL ANTIBODIES" CLINICA CHIMICA ACTA, Bd. 191, Nr. 3, 1990, Seiten 211-220, XP001036833 ISSN: 0009-8981
- ABRAHAMSON D R ET AL: "SELECTIVE IMMUNOREACTIVITIES OF KIDNEY BASEMENT MEMBRANES TO MONOCLONAL ANTIBODIES AGAINST LAMININ LOCALIZATION OF THE END OF THE LONG ARM AND THE SHORT ARMS TO DISCRETE MICRODOMAINS" JOURNAL OF CELL BIOLOGY, Bd. 109, Nr. 6 PART 2, 1989, Seiten 3477-3492, XP001036999 ISSN: 0021-9525
- YOKOYA YUKIHIRO ET AL: "Concentration of serum laminin and type IV collagen in liver disease assayed by a sandwich enzyme-immunoassay using monoclonal antibodies." CLINICA CHIMICA ACTA, Bd. 210, Nr. 1-2, 1992, Seiten 109-118, XP001036829 ISSN: 0009-8981
- MATTER MICHELLE L ET AL: "A novel laminin E8 cell adhesion site required for lung alveolar formation in vitro." JOURNAL OF CELL BIOLOGY, Bd. 124, Nr. 6, März 1994 (1994-03), Seiten 1083-1090, XP001036963 ISSN: 0021-9525
- KATAYAMA M ET AL: "URINARY LAMININ FRAGMENTS AS A TUMOUR MARKER POTENTIALLY REFLECTING BASEMENT MEMBRANE DESTRUCTION" BRITISH JOURNAL OF CANCER, Bd. 65, Nr. 4, 1992, Seiten 509-514, XP001036973 ISSN: 0007-0920
- DATABASE WPI Derwent Publications Ltd., London, GB; AN 1992-204526 XP002190739 & JP 04 134097 A (TAKARA SHUZO CO LTD), 7. Mai 1992 (1992-05-07)
- DATABASE WPI Derwent Publications Ltd., London, GB; AN 1991-098302 XP002190740 & JP 03 042572 A (TAKARA SHUZO CO LTD), 22. Februar 1991 (1991-02-22)
- DATABASE WPI Derwent Publications Ltd., London, GB; AN 1989-330803 XP002190741 & SU 1 454 851 A (A MED USSR ONKOLOGY), 30. Januar 1989 (1989-01-30)

## Beschreibung

Monoklonale Antikörper zur selektiven immunologischen Bestimmung von hochmolekularen, intakten Lamininformen in Körperflüssigkeiten

Die vorliegende Erfindung betrifft monoclonale Antikörper zur selektiven immunologischen Bestimmung von hochmolekularen Lamininformen in Körperflüssigkeiten sowie ein Verfahren zur Herstellung dieser Antikörper.

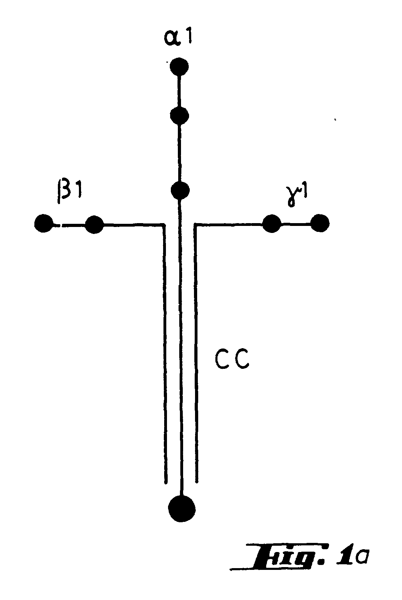

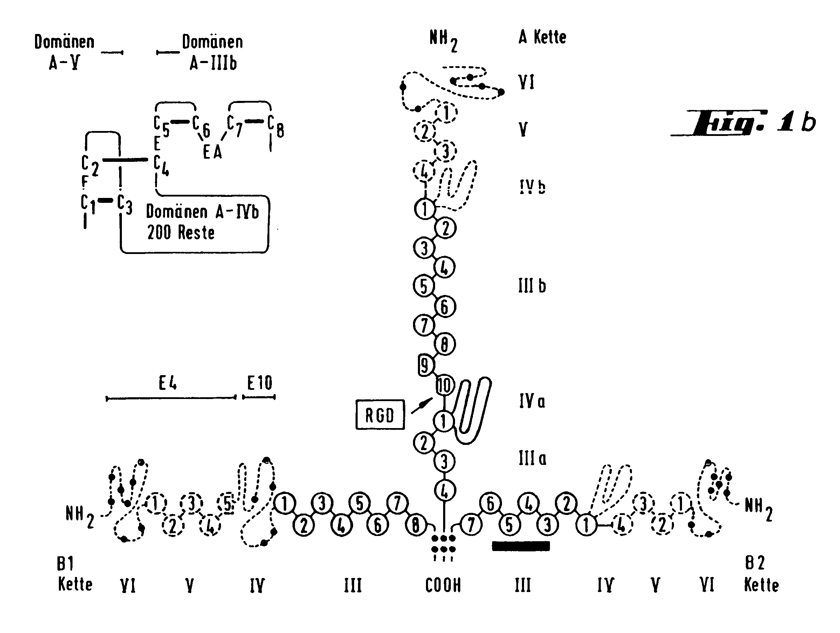

Laminin ist ein Multidomänen-Protein das in allen Basalmembranen gefunden wird und mit verschiedenen anderen typischen Komponenten der Basalmembran wie z.B. Nidogen, Heparansufat-Proteoglycan oder Kollagen IV komplexiert ist (Timpl, R. (1989) Eur. J. Biochem. 180; 487 bis 502). Es ist aus drei unterschiedlichen, disulfidverknüpften Polypeptiden zusammengesetzt. Dabei wird die Struktur eines asymmetrischen Kreuzes gebildet (Figuren 1a und 1b). In jüngster Zeit wurden viele Strukturvarianten identifiziert, die durch das Assembly von (derzeit bekannten) 8 verschiedenen Untereinheiten entstehen. Es müssen - um eine Lamininisoform zu erhalten - immer Polypeptide aus drei unterschiedlichen Molekülklassen assemblieren: eine α-Kette (früher A-Kette), eine β-Kette (früher B1-Kette) und eine γ-Kette (früher B2-Kette) (Burgeson, R.E.; et al. (1994) Matrix Biology, Vol. 14, 209 bis 211).
Den Lamininen werden eine Vielzahl interessanter biologischer Funktionen zugeordnet, wie z.B. Beeinflussung des Zellwachstums, des Zellspreadings und des Neuritenwachstums, sowie Induktion von Differenzierungsprozessen (Timpl, R. (1989) Eur. J. Biochem. 180; 487 bis 502).

Obwohl Laminine typische Komponenten aller Basalmembranen sind, zeichnen sich verschiedene Isoformen durch eine sehr spezifische Gewebsverteilung aus. Merosin z.B. (= Laminin 2; α2,β1,γ1) ist Bestandteil von Basalmembranen der Schwann-Zellen, gestreiften Muskeln und Trophoblasten (Leivo, I.; Engvall, E. (1988) Proc. Natl. Acad. Sci. USA 85; 1544 bis 1548).
Eine andere Variante, s-Laminin (= Laminin 3; α1,β2,γ1) ist in der Basalmembran von Synapsen an neuromuskulären Endplatten, im Endothel von Blutgefäßen und in glomerulären Podozyten anzutreffen (Hunter, D.D.; Shah, V.; Merlie, J.P.; Sanes, J.R. (1989) Nature 338; 229 bis 234).
Ein drittes Beispiel, K-Laminin (= Laminin 6; α3,β1/β2,γ1) ist spezifisch für die Basammembranen der Haut (Marinkovich, M.P.; Lunstrom, G.P.; Burgeson, R.E. (1992a) J. Biol. Chem. 267; 17900 bis 17906). Dort, als Komponente von "anchoring filaments", ist auch das für epitheliale Gewebe typische Kalinin/Nicein (= Laminin 5, α3,β3,γ2) anzutreffen (Marinkovich, M.P.; Lunstrom, G.P.; Keene, D.R., Burgeson, R.E. (1992b) J. Cell. Biol. 119; 695 bis 703).

Spezifische Bestimmungsmethoden für den Nachweis von Laminin in Humanserum wurden zur Diagnose verschiedener Erkrankungen entwickelt. Als mögliche Indikationen werden hepatische Fibrose/Zirrhose, alkoholische Leberfibrose, diabetische Komplikationen der BM, renale Erkrankungen, chronisch entzündliche Arthrose/chronische Polyarthritis, und Tumorerkrankungen genannt (Kropf. J.; et al. (1991) Clin Chem. 37; 30; Niemelä, O.; Risteli, L.; Sotaniemi, E.A.; Ristelli, J. (1985) Eur. J. Clin. Invest. 15; 132 bis 137; Katayama, M.; Kamihagi, K.; Hirai, S.; Murakami, K.; Hino, F.; Kate, I. (1992) Br. J. Cancer 65; 509 bis 514; Brocks, D.G.; Strecker, H.; Neubauer, H.P.; Timpl, R. (1986) Clin. Chem. 32; 787 bis 791; Horikoshi, S.; Koide, H. (1991) Clin. Chim. Acta 196; 185 bis 192).

Derzeit können drei Laminin-Bestimmungsmethoden kommerziell erworben werden:
Verfahren 1:
   Eine Laminin P1 Bestimmungsmethode auf der Grundlage eines polyclonalen Antiserums wird von der Behringwerke AG unter dem eingetragenen Warenzeichen "RIA-gnost^{®}" vertrieben (Brocks, D.G.; Strecker, H.; Neubauer, H.P.; Timpl, R. (1986) Clin. Chem. 32; 787 bis 791).
Verfahren 2:
   Ein Laminin EIA-Immunoassay auf Grundlage von zwei monoklonalen Antikörpern (TAKARA, Shuzo Co., LTD; Katayama et al., 1992; Katayama, M.; Kamihagi, K.; Hirai, S.; Murakami, K.; Hino, F.; Kate, I. (1992) Br. J. Cancer 65; 509 bis 514).
Verfahren 3:
   Ein one-step sandwich enzyme immunoassay, basierend auf zwei monoklonalen Antikörpern (Fuji Chemical Ltd.; Iwata, K. (1990) Clin. Chim. Acta 191; 211 bis 220).

Während in den Verfahren 1 und 3 Antikörper gegen das pepsinresistente Fragment "Lam P1" verwendet werden, basiert das Verfahren 2 auf Antikörpern gegen ein, nach der Methode Wewer et al. (schonender Pepsinverdau in Abwesenheit von EDTA; Wewer, U.; Albrechtsen, R.; Manthorpe, M.; Varon, S.; Engvall, E.; Rouslahti, E. (1983) J. Biol. Chem. 258; 12654 bis 12660) isoliertes, "natives Laminin":
Aus der Arbeit von Katayama (Verfahren 2) geht hervor, daß der TAKARA-Immunoassay relativ gut mit dem RIA-gnost^{®} (Behringwerke) korreliert (r = 0,68) obwohl eine zum RIA-gnost^{®} abweichende Antigenverteilung in einem (Lungen)Tumorserum vorliegt. Es werden nämlich nach einer Siebgelchromatographie von den gegen "natives Laminin" gerichteten Antikörpern im Serum zwei antigene Peaks im Mw-Bereich 330 bis 150 kDa nachgewiesen. Aufgrund der Größe muß es sich hierbei um Degradationsprodukte des "Serum-Laminins" handeln.

Der RIA-gnost^{®} (Verfahren 1) diagnostiziert im Gegensatz dazu zwei Peaks im Bereich 100 bis 900 kDa, also offensichtlich sowohl native (intakte) als auch degradierte Strukturen. Die gemeinsame Erkennung von intakten und degradierten Lamininstrukturen führt allerdings zu Ungenauigkeiten in der diagnostischen Aussage, da einerseits Normalkollektiv und Patientenkollektiv überlappen können, und andererseits Konzentrationsschwankungen in einem Peak durch gegenläufige Gehaltsverschiebungen im anderen Peak ausgeglichen werden können.
Für den Fuji-Immunoassay (Verfahren 3) liegt keine Chromatographie eines Serums vor. Aus einer SDS-Gelelektrophorese und Immunoblot wird aber ersichtlich, daß die verwendeten Antikörper im Normalserum und im "Leberzirrhose-Serum" eine Bande bei 200 kDa erkennen, also offensichtlich spezifisch mit degradierten Lamininfragmenten reagieren.

Iwata, K. beschreibt einen Einschritt-Sandwich-Enzymimmunoassay für humanes Laminin unter Verwendung von monoclonalen Antikörpern (Iwata, K. Clinica Chimica Acta. 191 (1990), 211-220). Monoclonale Antikörper gegen Laminin werden auch von Abrahamson D. R. et al. (1989 beschrieben (Abrahamson D. R. et al. J. Cell Biology (1989), 109, 3477-3491). Katayama et al. (1992) beschreiben des weiteren Laminin Fragmente als Tumormarker (Katayama, M. et al. Br. J. Cancer (1992), 62, 509-514).

Demgegenüber ist es Aufgabe der vorliegenden Erfindung, monoclonale Antikörper und ein Verfahren zu deren Hersellung bereitzusellen, welche bevorzugt an intaktem, nativem Laminin, insbesondere an den nativ gefalteten Strukturen der Laminin P1 Domäne des Laminins binden.

Der vorliegenden Erfindung liegt ferner die Aufgabe zugrunde, ein Verfahren zur immunologischen Bestimmung von Laminin auf Basis der zu entwickelnden Antikörper bereitzustellen, bei welchem ausschließlich die hochmolekulare Population des Laminins detektiert wird, wodurch unter Vermeidung der Miterfassung von Laminin-Degradationsprodukten eine genauere Bestimmung des Gehaltes von intaktem Laminin bzw. zweier Laminin-Subpopulationen in Körperflüssigkeiten ermöglicht wird. Damit werden bei einem Diagnoseverfahren, das auf einer derartigen Laminin-Bestimmungsmethode beruht, Ungenauigkeiten in der diagnostischen Aussage, so, wie sie bei den bisher bekannten Bestimmungsmethoden in Kauf genommen werden mußten, eliminiert.

Die Aufgabe wird erfindungsgemäß gelöst durch einen monoclonalen Antikörper
1. mit Spezifität zu Proteinen aus der Familie der Laminine und dem durch Pepsinverdau aus Humanplazenta herstellbaren Laminin P1 Fragment, der sich dadurch auszeichnet, daß der monoklonale Antikörper bevorzugt an den nativ gefalteten Strukturen der Laminin P1 Domäne des Laminins bindet, die Affinität des Antikörpers zum intakten, nativen Laminin ungefähr gleich der Affinität des Antikörpers zum Laminin P1 Fragment ist und der Antikörper die Eigenschaften eines monoklonalen Antikörpers aufweist, der von der Hybridoma-Zellinie DSM ACC2181, DSM ACC2180 oder DSM ACC2182 produziert wird,
2. welcher Antikörper sich vorzugsweise ferner dadurch auszeichnet, dass er durch ein Hybridom gebildet wird, das durch Fusion von Zellen aus einer Myelom-Zellinie und Lymphozyten von einem zuvor mit Laminin P1 immunisierter nicht-humanen Wirbeltier entstanden und anschließend danach ausgewählt worden ist, dass der gebildete Antikörper neben einer guten Bindungseigenschaft zum gereinigten Humanlaminin aus Plazenta auch eine gute Reaktion mit der hochmolekularen Form des aus Humanserum gewonnenen Laminins zeigt.
3. Der monoclonale Antikörper gemäß der vorliegenden Erfindung hat ferner vorzugsweise die Fähigkeit, paarweise mit einem weiteren erfindungsgemäßen Antikörper an das Antigen zu binden.

Die gestellte Aufgabe wird ferner gelöst durch eine Hybridoma-Zellinie, die einen Antikörper mit den unter Nr. 1 bis 3 dargestellten Eigenschaften produziert, herstellbar durch Fusion von Zellen aus einer Myelom-Zellinie und Lymphozyten von einem zuvor mit Laminin P1 immunisierter nicht-humanen Wirbeltier und nachfolgender Auswahl der Hybride danach, daß der von dem Hybrid produzierte Antikörper neben einer guten Bindungseigenschaft zum gereinigten Humanlaminin aus Plazenta auch eine gute Reaktion mit der hochmolekularen Form des aus Humanserum gewonnenen Laminins zeigt.

Eine solche Hybridoma-Zellinie zeichnet sich vorzugsweise dadurch aus, daß die Lymphozyten aus mit Laminin P1 immunisierten Mäusen des Balb/c-Stammes entnommen sind und die Myelom-Zellinie die Maus-Myelom-Zellinie P3X63AG8.653 ist.

Drei Hybridoma-Zellinien, welche die oben genannten Eigenschaften aufweisen, sind bei der Deutschen Sammlung von Mikroorganismen und Zellkulturen GmbH (DSM), Marscheroder Weg 1b, D-38124-Braunschweig, gemäß den Vorschriften des Budapester Vertrages am 12. Juli 1994 unter den Hinterlegungsnummern, DSM ACC2181, DSM ACC2180 und DSM ACC2182 hinterlegt worden.

Diese Hybridoma-Zellinien produzieren monoclonale Antikörper, welche die unter Nr. 1 bis 3 dargestellten, vorteilhaften Eigenschaften aufweisen.

Die Hybridoma-Zellinien DSM ACC2181 und DSM ACC2180 produzieren jeweils einen Antikörper der Subklasse IgG2a, die Hybridoma-Zellinie DSM ACC2182 einen Antikörper der Subklasse IG 1.

Zur Lösung der eingangs gestellten Aufgabe wird ferner ein Verfahren zur Herstellung eines erfindungsgemäßen monoclonalen Antikörpers bereitgestellt, daß sich dadurch auszeichnet, daß
a) nicht-humanen wirbeltiere mit dem durch Pepsinverdau von humaner Plazenta herstellbaren Laminin P1 Fragment immunisiert werden,
b) Lymphozyten der immunisierten Wirbeltiere gewonnen und mit Myelom-Zellen fusioniert werden,
c) die Hybride hinsichtlich des Vorliegens eines Antikörpers mit den unter Nr. 1 bis 4 angegebenen Eigenschaften ausgewählt und geklont werden und
d) der Antikörper aus diesen Klonen gewonnen wird.

Dieses Verfahren ist vorzugsweise dadurch gekennzeichnet, daß zur Ausführung des Verfahrensschritts d) die Hybridoma-Zellinien DSM ACC2181, DSM ACC2180 oder DSM ACC2182 eingesetzt werden.

Die gestellte Aufgabe wird darüber hinaus gelöst durch ein Verfahren zur immunologischen Bestimmung von nativem Laminin unter Verwendung eines adhäsiv oder kovalent an ein Trägermaterial fixierten Schichtantikörpers und eines markierten Zweitantikörpers, der das an den Schichtantikörper gebundene Antigen erkennt, dadurch gekennzeichnet, daß der Schichtantikörper ein monoclonaler Antikörper gemäß der vorliegenden Erfindung ist.

Vorzugsweise wird aber die gestellte Aufgabe gelöst durch ein Verfahren zur immunologischen Bestimmung von nativem Laminin unter Verwendung eines adhäsiv oder kovalent an ein Trägermaterial fixierten Schichtantikörpers und eines markierten Zweitantikörpers, der das an den Schichtantikörper gebundene Antigen erkennt, dadurch gekennzeichnet, daß der markierte Zweitantikörper ein monoclonaler Antikörper gemäß der vorliegenden Erfindung ist.

Dabei kann der Schichtantikörer ebenfalls ein monoclonaler Antikörper gemäß der vorliegenden Erfindung sein, vorzugsweise ist er derjenige, welcher von der Hybridoma-Zellinie DSM ACC2181 produziert wird und die in Tabelle 3 dargestellten Bindungskonstanten aufweist.
Die erfindungsgemäßen monoclonalen Antikörper können insbesondere Verwendung finden in Verfahren zur Diagnose von Erkrankungen, die mit einer Veränderung des Laminingehaltes in Körperflüssigkeiten einhergehen, wobei die Körperflüssigkeiten dem lebenden Körper entnommen, aber diesem nicht wieder zugeführt werden.

Im folgenden wird die Erfindung detailliert erläutert, insbesondere die bevorzugten Ausführungsformen. Ferner wird die Erfindung durch die Patentansprüche definiert.

Zur Herstellung der monoklonalen Antikörper können Tiere, bevorzugt Nagetiere, wie z.B. Mäuse, Ratten, Kaninchen, Meerschweinchen, mit Laminin P1, das nach der im Beispiel 1 beschriebenen Methode isoliert wurde, in Gegenwart von Adjuvans immunisiert werden.

Besonders bevorzugt werden Mäuse eingesetzt, insbesondere solche vom Balb/c Stamm. Mit wiederholten Sekundärinjektionen, beispielsweise im Abstand von 4 bis 8 Wochen, wird die Immunantwort verstärkt. Der Erfolg der Immunisierung wird durch Bestimmung der Konzentration von spezifischen Antikörpern mit einem, dem Fachmann an sich bekannten, ELISA kontrolliert. Einige Tage vor Fusion der Lymphozyten mit einer Myelom Zellinie werden die Tiere mit Laminin P1 ohne Adjuvans behandelt. Lymphozyten der Tiere werden gewonnen und mit einer Myelom Zellinie fusioniert, die ebenfalls von einer der oben genannten Tierspezies stammen kann, vorzugsweise jedoch von der Maus, insbesondere mit der Zellinie P3X63AG8.653. Es werden vorteilhaft Lymphozyten mit Myelom Zellinien gleicher Spezies fusioniert. Die Fusion und weitere Züchtung der Zellklone werden in einer dem Fachmann bekannten Weise durchgeführt, wobei im Überstand der Zellkultur mittels immunologischer Bindungstests die Konzentration spezifischer Antikörper bestimmt wird. Aus den aus der Fusion hervorgehenden Zellklonen werden geeignete Klone für die Verwendung in immunologischen Verfahren mit Hilfe einer, in den Tabellen 4 und 5 dargelegten, Screeningabfolge ausgewählt. Besonders bevorzugt wird mit Zellinien gearbeitet, die durch Fusion von Lymphozyten von Mäusen des Balb/c-Stammes gegen Laminin P1 mit der Maus Myelom-Zellinie P3X63AG8.653 hergestellt werden.

Die erfindungsgemäßen monoklonalen Antikörper gehören in die Gruppe der Immungloguline, bevorzugt in die Klasse der IgG-, IgA-, und IgM-Proteine. Antikörper der Subklasse IgG2a und IgG1 sind besonders vorteilhaft einsetzbar. Der erfindungsgemäße Antikörper zeichnet sich insbesondere dadurch aus, daß dessen Affinität zum intakten Laminin ungefähr gleich dessen Affinität zu dem durch Pepsinverdau erzeugten, Immunisierungsantigen Laminin P1 (Tabellen 2 und 3) ist. Die in den Tabellen 4 und 5 dargestellte Screeningstrategie verdeutlicht, daß tatsächlich die überwiegende Mehrheit der aus der Fusion gewonnenen Klone monoklonale Antikörper produziert, die lediglich das durch die Pepsinbehandlung erzeugte, artifizielle Laminin P1 Fragment ekennen. Diese Antikörper reagieren mit größter Wahrscheinlichkeit mit immunogenen Strukturen, die infolge proteolytischer Kettenspaltungen künstlich erzeugt wurden, die aber in der nativen Struktur des Proteins nicht vorliegen. Nur wenige der gewonnenen monoklonalen Antikörper, insbesondere aber die Antikörper gemäß der vorliegenden Erfindung, reagieren mit den nativen, nicht durch Pepsinverdau generierten Strukturen in der zentralen Domäne des Laminins.

Für die Herstellung und Charakterisierung der erfindungsgemäßen Antikörper ist es wichtig, daß eine geeignete Quelle zur Gewinnung des Immunisierungsantigens und der verschiedenen, für das Screening entscheidenden Strukturvarianten zur Verfügung steht. Humanes Laminin P1 sowie unterschiedliche Lamininpräparate werden nach den in den Beispielen beschriebenen Methoden aus humaner Plazenta gereinigt. Aus diesem Organ können wenigstens zwei unterschiedliche Lamininisoformen gewonnen werden (Brown, C.J.; Wiedemann, H.; Timpl, R. (1994) J. Cell Sci. 107; 329 bis 338).

Die erfindungsgemäßen Antikörper können in verschiedenen immunologischen Verfahren, wie z.B. immunoradiometrischen Tests nach Markierung des Tracerantikörpers mit Chloramin T oder Bolton-Hunter-Reagenz, sowie anderen kompetitiven und nicht-kornpetitiven Bindungsassays, wie Fluoreszenz-, Enzym-, Chemilumineszenz- oder anderen Immunoassays, einschließlich allen Formen des Radioimmunassays verwendet werden (Harlow, E.; Lane, d. (1988) Antibodies: A Laboratory Manual, CSH; 2nd Edn.; New York; 319 bis 359; 553 bis 612). Dabei ist es unerheblich, ob der Schichtantikörper an Polystyrolröhrchen, Polystyrolkügelchen, paramagnetische Partikel oder aktivierte Säulenmaterialien aller Art kovalent oder nicht-kovalent gebunden ist. Die monoklonalen Antikörper können daher in immunologischen Verfahren zur Isolierung und Charakterisierung sowie zur quantitativen Bestimmung von Laminin in Geweben und Körperflüssigkeiten eingesetzt werden. Man verfährt nach den dem Fachmann an sich bekannten Methoden, indem eine flüssige Probe, die Laminin enthält, mit einem der erfindungsgemäßen monoklonalen Antikörper, sei es in Lösung oder vorzugsweise auf einem festen Träger zur Reaktion gebracht wird und die Menge des Laminins über den gebildeten Antigen-Antikörper-Komplex bestimmt wird. Die bislang bei der immunologischen Bestimmung miterfaßten, bzw. ausschließlich erkannten Degradationsprodukte des Laminins in Körperflüssigkeiten werden bei diesen Immunoassays nicht erkannt, sondern ausschließlich intakte Lamininstrukturen.

In den folgenden Beispielen wird die Erfindung weitergehend erläutert.

### Beispiel 1

### Herstellung von humanem Laminin P1 (Immunisierungsantigen)

Das Immunisierungsantigen Laminin P1 ist ein 200 bis 250 kDa großes Fragment des Laminins das nach intensivem Pepsinverdau von Plazentagewebe extrahiert und isoliert werden kann. Dieses Fragment enthält die inneren stäbchenförmigen Domänen (Domänen III) der kurzen Arme der Lamininketten α, β und γ (Risteli, L; Timpl, R. (1981) Biochem. J. 193, 749 bis 755). Die drei Polypeptidketten (von denen mehrere Isoformen existieren) sind durch Disulfidbrücken verknüpft und zeichnen sich durch eine identische dreidimensionele Struktur aus. Wie in Abbildung 1b dargestellt, liegen in den einzelnen Kettenabschnitten der Lamininuntereinheiten sieben bis vierzehn linear aneinander gereihte Strukturmotive, sog. EGF-like repeats vor, die alle durch dasselbe Faltungsmuster charakterisiert sind. Die jeweils 50 bis 60 Aminosäuren eines EGF-like repeats nehmen durch eine festgelegte typische Reihenfolge von Disulfidverknüpfungen eine kleeblattförmige Struktur an. Auf Sequenzebene liegt zwischen den EGF-like repeats der kurzen Lamininketten eine Identität von > 30 % vor (Engel, J. (1989) FEBS Lett. 251, No. 1,2; 1 bis 7).

Das Immunisierungsantigen Laminin P1 wurde wie nachfolgend erläutert hergestellt:
1. Auftauen
   1,2 kg Humanplazenta wurden in 500 ml Wasser + PI bei 4°C 16 bis 20 Stunden aufgetaut.
   (PI = Protease Inhibitoren = 1mM NEM, 1mM PMSF, 0,28mM PCMB)
2. Homogenisierung
   Nach dem Auftauen wurde das Volumen im Ansatz auf 2,5 l eingestellt und mit dem Ultraturrax 3 bis 5 Minuten homogenisiert.
   Schließlich wurde bei 6500 x g 10 Minuten bei 4°C zentrifugiert.
3. Waschen
   Der gewonnene Niederschlag wurde 8 x mit je 1,8l 3M NaCl, 0,1 % Triton X-100, 0,02M Tris/HCl pH 7,4 + PI aufgerührt und anschließend bei 6500 x g, 10 Minuten, 4°C zentrifugiert. Abschließend wurde der Niederschlag in Wasser gerührt und erneut zentrifugiert. Der Niederschlag wurde bei 4°C 64 Stunden mit 3L Puffer (1M NaCl, 10mM EDTA, 1 % Triton X-100, 0,02 M Tris/HCl pH 8,6 + PI) extahiert. Nicht lösliches Material wurde durch Zentrifugation bei 26000 x g (30 Minuten) abgetrennt und weiterverarbeitet.
4. Pepsinverdau
   Der letzte Niederschlag wurde in 3,0l 0,5M Essigsäure, 10mM EDTA aufgerührt, mit dem Ultraturrax 3 Minuten homogenisiert und weitere 2 Stunden gerührt. Der pH-Wert des Ansatzes wurde dann mit Ameisensäure auf 2,5 eingestellt. Die proteolytische Verdauung erfolgte nach Zugabe von 100 mg Pepsin bei 4°C und dauerte 40 h. Nach dem Pepsinverdau wurden vorhandene Trübungen durch 30 minütiges Zentrifugieren bei 26000 x g (4°C) entfernt.
5. Saure NaCl Fällung
   In den Überstand der Zentrifugation (3,16 l) wurden 366,1 g NaCl unter Rühren eingtragen (= 1,9M) und weitere 2 Stunden gerührt.
   Das gebildete Präzipitat wurde durch Zentrifugation bei 6500 x g (4°C, 10 min) vom sauren Puffer getrennt, in 1,8L 0,02M NaCl, 2M Harnstoff, 0,05M Tris/HCl pH 8,6 gelöst und 7x gegen 8L des gleichen Puffers dialysiert. Trübungen in der Lösung konnten durch Zentrifugation (30 Minunten, 26000 x g, 4°C) entfernt werden.
6.Chromatographie an Q-Sepharose FF (5x15 cm)
   Es wurden 5 Trennungen durchgeführt und jeweils 350 ml Probe auf die Säule aufgetragen.
   - Laufbedingungen:: Puffer A; 0,02M NaCl, 0,05M Tris/HCl, 1 mM EDTA pH 7,4
   Puffer B; 1 M NaCl, 0,05M Tris/HCl, 1 mM EDTA pH 7,4 flow rate; 3 ml/min
   linearer Gradient (90 ml) von 0,02 auf 0,1 M NaCl
   Stufengradient; 300 ml Puffer mit 0,1 M NaCl
   450 ml Puffer mit 0,25M NaCl
   300 ml Puffer mit 0,5M NaCl

   Die einzelnen Fraktionen der Chromatographie wurden mit Hilfe des RIA-gnost^{®} Laminin P1 analysiert und die Laminin P1 enthaltenden Fraktionen (Elution bei 0,25M NaCl) wurden gepoolt.
7. Ammoniumsulphatfällung
   Der Laminin P1 enthaltende Pool wurde 3:1 mit 3M (NH₄)₂S0₄ verdünnt und 2 Stunden bei 4°C inkubiert. Das Präzipitat wurde durch 30 minütige Zentrifugation bei 26000 x g (4°C) gewonnen.
8. Chromatographie an Superose 6 prep grade 16/50
   Das gefällte Protein wurde in einem geeigneten Volumen 50mM Na-phosphat, 0,15M NaCl, 0,02 % Na-azid pH 2,0 gelöst und in 2 ml Portionen über Siebgelchromatographie getrennt.
   - Laufbedingungen:: Puffer; 50mM Na-phosphat, 0,15M NaCl, 0,02 % Na-azid
   pH 2,0
   flow rate; 1,0 ml/min

   Die einzelnen Fraktionen der Chromatographie wurden wieder mit Hilfe des RIA-gnost^{®} Laminin P1 analysiert. Die Laminin enthaltenden Fraktionen der 25 separaten Chromatographien wurden vereinigt und mit 2M NaOH versetzt, so daß sich ein pH-Wert von 8,0 einstellte. Die Lösung wurde dann 3:1 mit einer 3M (NH₄)₂S0₄ Lösung versetzt und über Nacht inkubiert. Das gebildete Präzipitat wurde bei 26000 x g (30 Minuten, 4°C) abzentrifugiert und in 20 mL 0,2M NH₄HCO₃ gelöst.
9. Kollagenaseverdauung
   Zu der Lösung wurden ca. 1 mg Kollagenase, sowie 1 Spatelspitze MgCl₂ und CaCl₂ zugegeben und 4 Stunden bei 37°C inkubiert. Der proteolytische Verdau wurde durch Zugabe von 3 ml Ameisensäure abgestoppt.
10. Chromatographie an Superose 6 prep grade 16/50
   Nach dem Verdau wurde erneut wie oben beschrieben chromatographiert.
   Die im RIA positiven Fraktionen wurden (nachdem mit 2M NaOH ein pH von 8,0 engestellt war) mit 3M (NH₄)₂S0₄ 3:1 verdünnt und in dieser Form bei 4°C aufbewahrt.
   Der gebildete Niederschlag wurde in 3M (NH₄)₂SO₄ suspendiert in 22 Portionen ä 0,5 mL vereinzelt und in der Eppendorfzentrifuge 4 Minuten zentrifugiert. Vom Überstand der Aliquots wurden jeweils 0,48 mL abgenommen.
   Das isolierte Laminin P1 kann als Ammoniumsulfat-Präzipitat bei 4°C wenigstens 4 Monate ohne Gehaltsverlust aufbewahrt werden.
11. Ausbeute/Qualität
   Konzentrationsbestimmung mit RIA-gnost^{®} Laminin P1:
   108 600 E (23,9 mg)
   Charakteristik im RIA:
   lineare Inhibitionskurve
   So-Bindung = 66,9 %
   50%-Intercept = 1,302 E/ml
   Normalserum = 1,72 E/ml

### Beispiel 2

### Herstellung von Laminin Charge I

Die Waschschritte und Extaktion erfolgen wie in Beispiel 1 beschrieben.

Das extrahierte Protein wird mit einer Ultrasette (300 kDa) in 2 M Harnstoff, 0,05 M Tris/HCl, 0,02 M NaCl, 2 mM EDTA, pH 7,4 umgepuffert und auf eine Q-Sepharose FF 60/14 aufgetragen. Das gebundene Protein kann bei 0,15 M NaCl, gelöst im Auftragspuffer, eluiert werden. Das Eluat wird wieder mit der Ultrasette (300kDa) konzentriert und über eine Superose 6 prep. grade 16/50 bei einer Flußrate von 1 ml/min in PBS, 2 mM EDTA chromatographiert. Die Laminin enthaltenden Fraktionen (mit dem RIA-gnost^{®} Laminin P1 bestimmt) werden vereint, mit der Ultrasette konzentriert und bei RT, mit 5000 Units Benzonase (purity II) > 2h inkubiert. Anschließend wird die Probe auf eine mit 0,15 M NaCl, 0,05 M Tris/HCl, pH 7,4 equilibrierte ConA-Sepharose 4B (2,6 x 10 cm) aufgetragen. Bei einer Flußrate von 1 ml/min kann das Laminin mit 0,4 M Methyl α-D-Mannopyranosid im Equilibrierungspuffer wieder eluiert werden. Das Eluat wird zur weiteren Reinigung und zwecks Überführung in einen PBS + 2 mM EDTA-Puffer mit einer Sephacryl S500 Superfine (2,6 x 140 cm, 1 ml/min) chromatographiert.

### Beispiel 3

### Herstellung von Laminin Charge II

Die Waschschritte und Extaktion erfolgen wie in Beispiel 1 beschrieben.

Das Laminin enthaltende EDTA-Extrakt wird mit Hilfe einer Ultrasette (300kDa) auf 25 ml konzentriert und gleichzeitig in 50 mM Tris/HCl 1 mM MgCl₂ pH 8,0 umgepuffert. Anschließend wird mit 5000 Units Benzonase 2 Stunden bei Raumtemperatur inkubiert. Die so behandelte Lösung wird dann über eine anti MAK P1 Affinitätssäule geleitet , an die 4 verschiedene monoclonale Antikörper mit Bindungseigenschafter, sowohl zum Laminin P1 als auch zu intaktem Laminin kovalent (nach den Angaben des Herstellers) gekoppelt wurden. Folgende von uns zur Unterscheidung so bezeichnete monoklonale Antikörper wurden an einer aktivierten CNBr-Sepharose 4B (6 ml) immobilisiert:
A24/2/2 (2,7 mg), A 27/2/1 (1,5 mg), A9/2/1 (0,6 mg) und A 28/1/1 (5,2 mg).

Auf die Affinitätssäule (equilibriert in 0,1 M NaCl, 0,05 M Tris/HCl, 10 mM EDTA, 0,1 M Pefabloc, pH 7,4) werden 25 ml EDTA-Extrakt bei einer Flußrate von 1 ml/min aufgetragen und dann mit 0,1 M Glycin/HCl pH 2,7 eluiert. Der pH-Wert des Eluats muß sofort mit Hilfe einer 0,8 M Trislösung neutral gestellt werden, abschließend wird mit Hilfe eines Macrosep 100 kDa in 0,1 M NH₄HCO₃ + 2 mM EDTA umgepuffert.

### Beispiel 4

### Hybridoma Produktion

Mäuse vom Balb/c-Stamm werden mit 20 µg Laminin P1 das nach Beispiel 1 erhalten wurde, in Gegenwart von komplettem Freund'schem Adjuvans subcutan immunisiert. Nach 4 Wochen und nach drei Monaten wird die Immunreaktion durch eine weitere subcutane Injektion von 20 µg Laminin P1 in Gegenwart von inkomplettem Freund'schen Adjuvans verstärkt. Drei Tage vor der Fusion wird die Immunantwort durch intraperitoneale Injektion von weiteren 100 *µ*g Laminin P1 verstärkt.

Zur Fusion werden die Tiere getötet und die Milzzellen isoliert. In Gegenwart von Polyethylenglycol werden die Milzzellen mit der Myeloma Zellinie P3X63AG8.653 fusioniert. Durch Kultivierung der Fusionsmischung in Hypoxanthin-Aminopterin-Thymidin-Medium über einen Zeitraum von zwei Wochen wird auf Milzzell x P3X63AG8.653-Hybride selektioniert. Zur Erreichung einer stabilen Ziellinie werden die erhaltenen Zellklone mehrfach subkloniert. Die entstehenden Zellkolonien werden in verschiedenen immunologischen Bindungstests auf Antikörperproduktion getestet. Die entstandenen Zellinien aus denen man die von uns zur Unterscheidung benannten Antikörper A27/2/1, A9/2/1 und A33/2/20 gewinnt sind bei der Deutschen Sammlung von Mikroorganismen und Zellkulturen GmbH (DSM), Merscheroder Weg 1b, D-38124 Braunschweig, gemäß den Vorschriften des Budapester Vertrags am 12. Juli 1994 unter den Hinterlegungsnummern DSM ACC2181, DSM ACC2180 und DSM ACC2182 hinterlegt worden.

### Beispiel 5

Versuche zur Charakterisierung und Identifizierung von spezifischen monoklonalen Antikörpern

Eine chronologische Abfolge des Screenings ist in den Tabellen 4 und 5 dargestellt.

Die Immunisierung von Mäusen mit Laminin P1 führt zu einer außerordentlich großen Anzahl, Antikörper produzierender Hybridoma Klone. Um nun Klone zu finden, die monoklonale Antikörper gegen Strukturmotive produzieren, welche in der entsprechenden Domäne des nativen Lamininmoleküls vorliegen, mußte ein differenzielles Screening durchgeführt werden, bei dem mit den unterschiedlichsten immunologischen Analysemethoden die Bindung der monoklonalen Antikörper an native bzw. natürliche Strukturen detektiert werden kann. Antikörper, die nur mit dem gereinigten Laminin P1 reagieren, wurden sofort ausgesondert.
Für die Durchführung der Experimente muß natives Laminin durch Extraktion aus Humanplazenta gewonnen werden (sh. Beispiele 2 und 3). Es muß dabei aber darauf geachtet werden, daß durch eine zu weitgehende Aufreinigung einer dominanten Lamininform das Spektrum der möglicherweise vorliegenden Lamininisoformen nicht zu weit eingeschränkt wird. Aus einer Arbeit von Brown et al. (Brown, C.J.; Wiedemann, H.; Timpl, R. (1994) J. Cell Sci. 107; 329 bis 338) geht hervor, daß zumindest zwei unterschiedliche Lamininvarianten (Laminin 2 und Laminin 4) aus Plazenta gewonnen werden können.
Als äußerst wichtig erschien es schon in frühen Phasen des Screeningprozesses die Reaktion der monoklonalen Antikörper mit dem im Serum vorliegenden Lamininstrukturen zu testen. Für diese Untersuchungen muß Serumlaminin mit Hilfe einer Sephadex S-400 Säule (1,0 x 30 cm) aus ca. 20 ml Serum von gesunden Probanden isoliert werden. Die Serumantigene eluieren in zwei breiten Peaks von der Säule, wobei Peak 1 antigene Strukturen mit Molekulargewichten > 600 kDa enthält. Der Peak 2 beinhaltet Degradationsprodukte des Serumlaminins mit Molekulargewichten in der Größenordnung des Immunisierungsantigens (ca. 200 kDa), bzw. kleinere Fragmente. Im Verlauf des Screenings werden nur die Antikörper (Klone) selektioniert, die neben ihrer Bindungseigenschaft zum gereinigten Humanlaminin aus Plazenta auch eine gute Reaktion mit dem "Serumlaminin", bevorzugt mit der hochmolekularen Form, zeigen. Ein weiteres Auswahlkriterium ist die Fähigkeit paarweise mit einem zweiten monoklonalen Antikörper an das Antigen zu binden. In Tabelle 6 ist am Beispiel von einigen monoklonalen Antikörpern der gleichen Immunisierug demonstriert, wie die einzelnen "Screeningbausteine" (sh. Tabelle 4 und 5) in ihrer Zusammenschau eine Charakterisierung und Selektion der erfindungsgemäßen monoklonalen Antikörper ermöglicht. Die Tabelle 7 faßt Untersuchungen zusammen, die darauf abzielten die Bindungseigenschaften zum Serumlaminin im Vergleich zur Standarderkennung zu analysieren.

### Beispiel 6

### Radioaktive Markierung der monoklonalen Antikörper A 27/2/1 und A 9/2/1

0,2 ml einer Lösung mit 40 µg der monoklonalen Antikörper in 0,05 M Phosphatpuffer pH 7,4 werden in einem Polystyrol-Teströhrchen (12 x 55 mm) vorgelegt und mit 100 MBq Na ¹²⁵J-Lösung, abgepuffert mit 0,5 M Phosphatpuffer pH 7,4, versetzt. Nach Zusatz von 50 µl einer wäßrigen Lösung von 20 µg Chloramin T wird 1 min gemischt. Anschließend wird die Jodierungsreaktion durch Zugabe von 50 *µ*l einer wäßrigen Lösung von 20 *µ*g Natriumdisulfit beendet.

Das nicht umgesetzte Na ¹²⁵J wird anschließend von den ¹²⁹J-markierten monoklonalen Antikörpern durch Chromatographie an einem Anionenaustauscher oder einer PD-10 Siebgelchromatographie abgetrennt. Die aufgereinigten ¹²⁵J-markierten Antikörper haben eine spezifische Aktivität von 5 bis 12 mCi/mg (180 bis 450 MBq/mg).

### Beispiel 7

### Antikörperbeschichtung der Teströhrchen

Zur Fixierung des monoclonalen Antikörpers A27/2/1 auf Polystyrolteströhrchen (12 x 75 mm) werden in jedes Röhrchen 0,5 ml des monoclonalen Antikörpers A27/2/1 bei einer Konzentration von 20 µg/ml in PBS 20 Stunden bei RT inkubiert. Nach Absaugen der Antikörperlösung wird mit 1 ml PBS/1 % BSA 1 Stunde bei RT blockiert. Die Röhrchen können nach dem Absaugen der Lösung im Kühlschrank aufbewahrt werden.

### Beispiel 8

### Immunradiometrische Tests

### Assayvariante 1: A27/2/1 - ¹²⁵J A9/2/1

In die beschichteten Röhrchen werden bei 17 bis 25°C je 50 µl Probe oder 100 µl Standard pipettiert und mit 150 µl PBS/Tween aufgefüllt. Nach 2 Stunden Inkubation wird abgesaugt und 2x gewaschen. Anschließend werden 200 µl ¹²⁵J A9/2/1 zugegeben und erneut 2 Stunden bei RT inkubiert. Nach dem Absaugen und zwei Waschschritten kann die gebundene Aktivität im γ-counter bestimmt werden.

### Assayvariante 2: A27/2/1 - ¹²⁵J A33/2/20

In die beschichteten Röhrchen werden bei 17 bis 25°C je 200 µl Probe oder Standard pipettiert. Anschließend werden 200 *µ*l Tracer zugegeben und 4 Stunden bei RT inkubiert. Nach dem Absaugen und zwei Waschschritten kann die gebundene Aktivität im γ-counter bestimmt werden.

### Standardantigen

Als Standard wird Laminin-P1 aus dem RIA-gnost^{®} Lam-P1 Kit (Behringwerke AG, Marburg) verwendet. Damit haben die beiden Assays eine gemeinsame Bezugsgröße und können gut miteinander und mit dem RIA-gnost^{®} Lam-P1 verglichen werden.

### Beispiel 9

Bestimmung der Molekulargewichtsverteilung der mit den beschriebenen Testverfahren reagierenden Standardproben und Antigene in Normalseren sowie pathologischen Seren

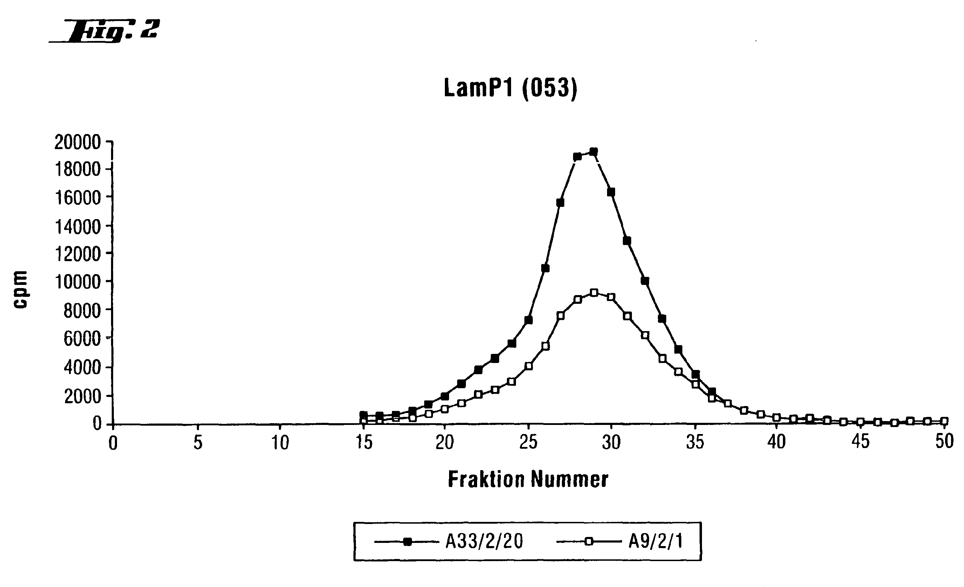

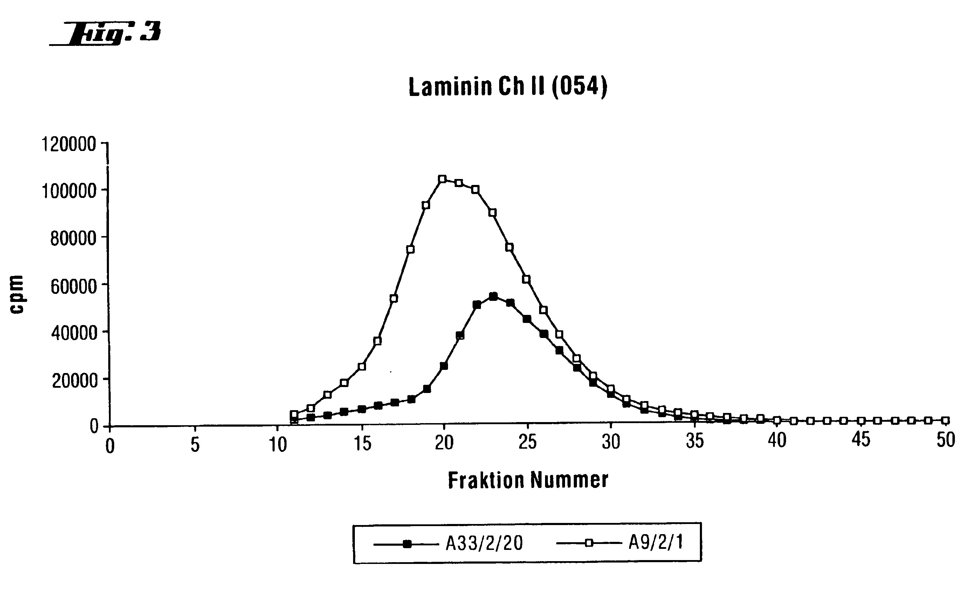

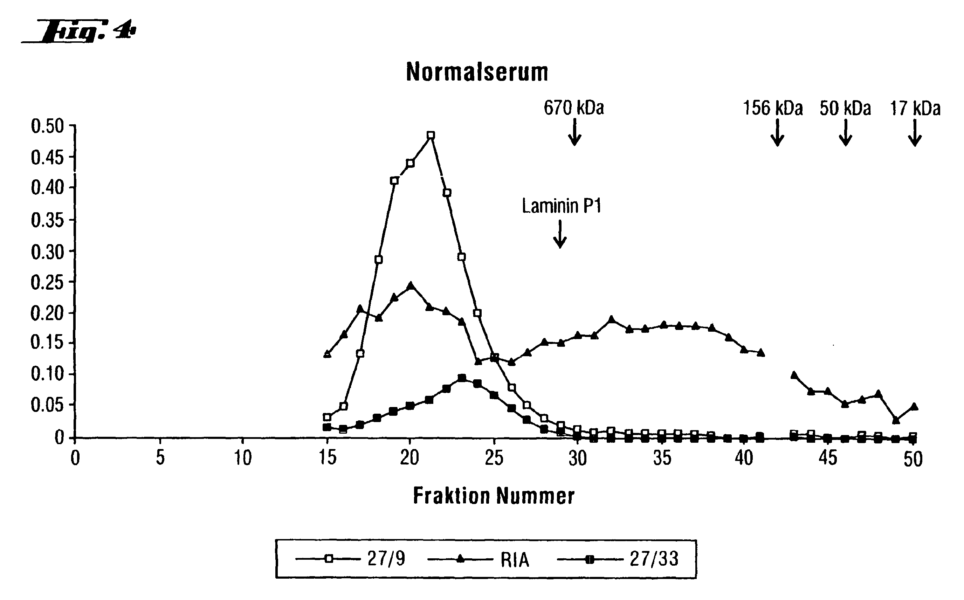

Laminin P1, Humanlaminin (Charge II) sowie verschiedene Seren werden mit Hilfe einer Sepharose S-400 Säule nach Molekulargewicht fraktioniert. Die Größenverteilung der Lamininantigenität wird dann mit Hilfe der immunoradiometrischen Testverfahren untersucht. Im Falle eines Normalserumpools werden die Ergebnisse mit dem RIA-gnost^{®} Lam P1 verglichen. In den Figuren 2 bis 4 sind die Chromatogramme der Standards und eines Normalserumpools gezeigt.

| | |
|---|---|
| Säulendimension: | 1,0 x 30 cm |
| Laufpuffer: | PBS + 0,04 % Tween-20 + 0,02 % Na-azid |
| Flußrate: | 0,2 ml/min |

Die Eichung der Säule erfolgte mit den Molekulargewichtsmarkern Thyroglobulin (670 kDa), Immunglobulin (156 kDa), Ovalbumin (44 kDa) und Myoglobin (17 kDa).

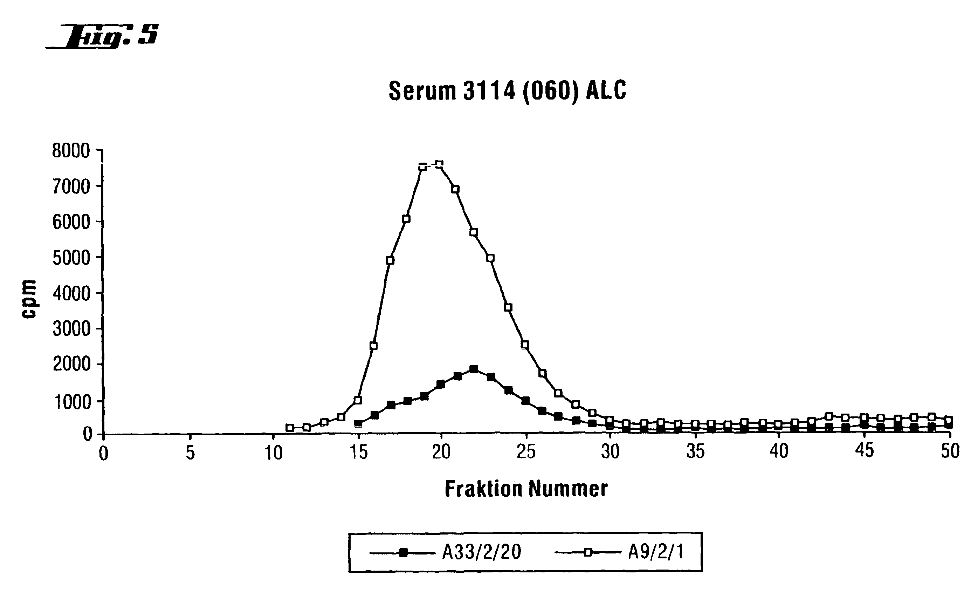

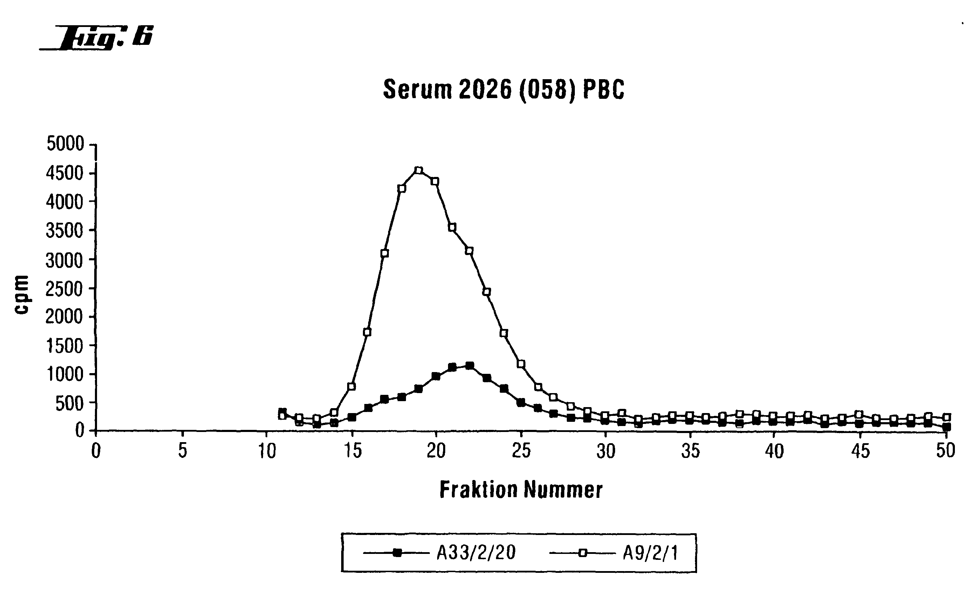

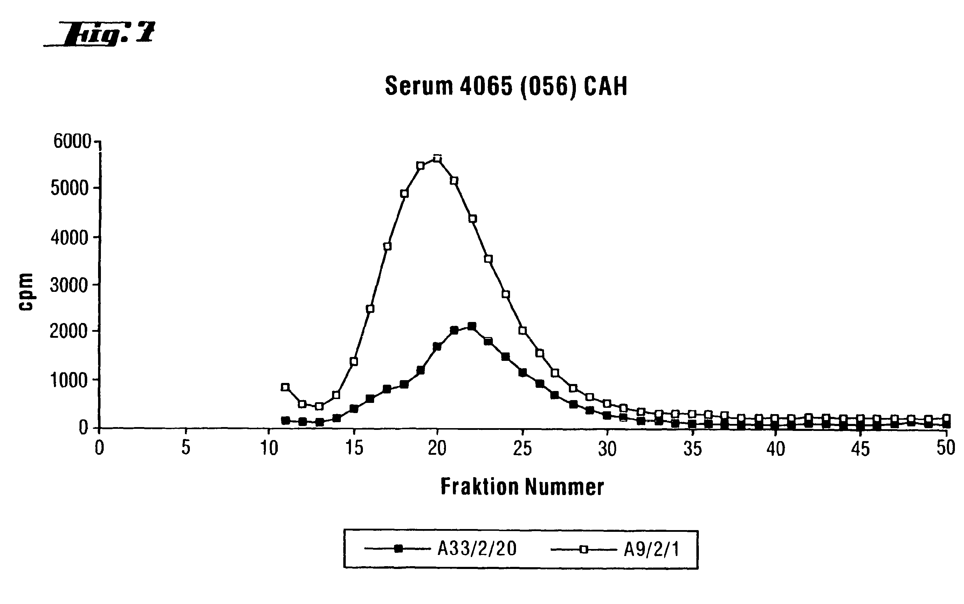

In den Figuren 5 bis 7 sind Einzelseren von Patienten mit Alkoholischer Lebererkrankung, PBC, und CAH dargestellt.

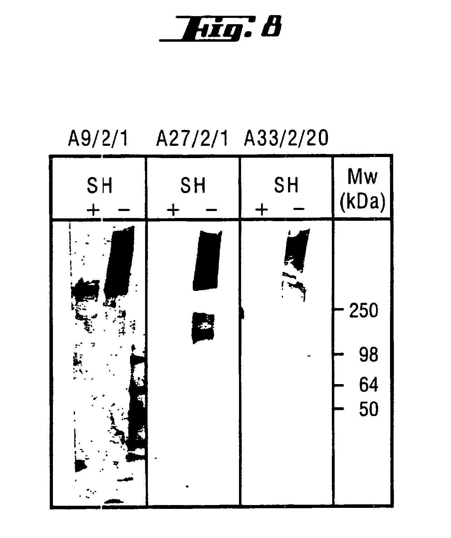

Figur 8 zeigt einen Semidry Blot nach Standardvorschrift mit diskontinuierlichem Puffersystem nach Trennung von ca. 1 µg Laminin Charge II (Beispiel 3) durch SDS-Gelelektrophorese (Novex Fertiggel 4 bis 12 % Polyacrylamid) unter reduzierenden (+ SH) bzw. nicht-reduzierenden Bedingungen (-SH). Die Nitrozellulosemembran wurde in Streifen geschnitten, die einzelnen Streifen wurden mit den monoklonalen Antikörpern A9/2/1, A27/2/1 und A33/2/20 inkubiert. Als Zweitantikörper wurde anti-Maus alk. Phosphatase (Sigma A 5153) verwendet.

Es wird deutlich, daß mit den erfindungsgemäßen Assays typische und jeweils charakteristische Antigen-Verteilungsmuster aufgezeichnet werden. Dies ist ein deutlicher Hinweis, daß die beiden immunologischen Bestimmungsverfahren unterschiedliche Lamininformen spezifisch erkennen.

Darüberhinaus ist zu erkennen, daß die monoclonalen Antikörper keine Reaktion mit den durch Reduktion erzeugten, denaturierten Strukturen aufweisen. Demzufolge erkennen die monoclonalen Antikörper gemäß der vorliegenden Erfindung spezifisch nativ gefaltete Strukturmotive der Laminin-P1-Domäne.

### Beispiel 10

Bestimmung der Bindungseigenschaften zu verschiedenen Laminin/Laminin P1 Präparationen

Um andeutungsweise einschätzen zu können welche Lamininstrukturen von den zwei Assays erkannt werden, wurden verschiedene Lamininpräparationen untersucht. Die folgende Tabelle erlaubt zwar keine eindeutigen Zuordnungen, sie zeigt aber deutlich, daß die zwei Tests mit unterschiedlichen Prioritäten (Affinitäten) bestimmte Varianten binden.
Die Frage welche Isoformen des Laminins durch die beschriebenen Testmethoden erkannt werden kann durch die gezeigten Daten nicht geklärt werden. Eine Lösung dieses Problems wäre nur dann möglich, wenn mehrere Isoformen (in ihrer nativen Form) gereinigt vorliegen würden.

| Konzentrationsbestimmung in %, bezogen auf die Gesamt-Proteinkonzentration der Probe | A27/A9 | A27/A33 |
|---|---|---|
| Laminin (Chemicon; Wewer 1983) *); | 2,7 | 1,4 |
| Merosin (Chemicon; Ehrig 1990) **); | 35,8 | 13,3 |
| Humanlaminin, Reinigung sh. Beispiel 2 | 21,4 | 2,8 |
| Humanlaminin, Reinigung sh. Beispiel 3 | 59,8 | 80,4 |
| Lam-P1: RIA-gnost^{®} Standard 7; % Bindung | | |
| bezogen auf die eingesetzten counts | 64,9 % | 35,4 % |

| | | |
|---|---|---|
| *) Wewer, U.; Albrechtsen, R.; Manthorpe, M.; Varon, S.; Engvall, E.; Rouslahti, E. (1983) J. Biol. Chem. 258; 12654 bis 12660) **) Ehrig, K.; Leivo, 1.; Argraves, S.W.; Ruoslahti, E.; Engvall, E. (1990); Proc. Natl. Acad: Sci. USA 87; 3264 bis 3268) | | |

### Beispiel 11

### Kreuzreaktionen

Tabelle 8 zeigt, daß bei den zwei neuen Laminin-Assays keine nennenswerten Kreuzreaktionen zu ausgewählten humanen Bindegewebs- und Serumproteinen nachweisbar sind. Ebenso kann keine Kreuzreaktion zu Laminin/Nidogen und Laminin P1 aus dem EHS-Tumor der Maus festgestellt werden.

### Beispiel 12

Bestimmung der durchschnittlichen Serumgehalte in Patientenkollektiven mit unterschiedlichen Erkrankungen

Verschiedene Serumkollektive wurden gemäß der Vorschriften in Beispiel 8 mit Hilfe der erfindungsgemäßen immunoradiometrischen Assays analysiert. Die Ergebnisse der Gehaltsbestimmungen sind in den Tabellen 9 bis 17 zusammengefaßt. Beide Testvarianten diagnostizieren in den Indikationen Alkoholische Lebererkrankungen, PBC, CAH, Posthepatische Leberzirrhose, Dekompensierte Leberzirrhose, Leberzirrhose unbekannten Ursprungs sowie in Tumorseren erhöhte Lamininspiegel, in der Indikation Diabetes werden deutlich erniedrigte Laminingehalte gefunden. Anhand der in den Tabellen aufgezeigten Korrelationen der erfindungsgemäßen Assays zum RIA-gnost^{®} Laminin P1 wird deutlich, daß sich beide immunologische Verfahren voneinander unterscheiden und daß beide Assays in ihrer diagnostischen Aussage vom RIA-gnost^{®} abweichen. Damit wird der in Beispiel 9 beschriebene Effekt - ein Unterschied im Antigen - Verteilungsmuster - bestätigt.

Abkürzungen
- EDTA:: Ethylendiamin-tetraessigsäure
- NEM:: N-Ethylmaleinimid
- PBS:: Phosphate buffered saline (Buffer solution PM 16, Serva)
- PCMB:: 4-Hydroxymercuri-benzoesäure Natriumsalz
- PMSF:: Phenylmethylsulfonylfluorid

Chemikalien, Enzyme
Kollagenase, Worthington (CLSPA)
Pepsin, Boehringer Mannheim (Nr. 108057)
Benzonase, Merck Darmstadt (Nr. 1654)

Alle verwendeten Chemikalien waren mit "p.A." spezifiziert; sie wurden von den Firmen Riedel d.H., und Merck bezogen.

Trennmedien
Q-Sepharose^{®} FF, Pharmacia
Superose^{®} 6 (16/50), Pharmacia
Ultrasette^{®} (300 kDa), Filtron
BrCN-aktivierte Sepharose^{®}, Pharmacia
ConA-Sepharose^{®} 4B, Pharmacia
Sephadex^{®} S-400, Pharmacia
Erläuterungen zu den Tabellen 1 bis 3:

BIAcore Untersuchungen
Mit dem BIAcore^{®}-System der Firma Pharmacia Biosensor können biospezifische Interaktionen on-line verfolgt werden. Das Prinzip der Messung beruht auf einem optischen Phänomen (surface plasmon resonance), das durch die auf einem Goldfilm gebundene Masse beeinflußt wird. Vereinfacht ausgedrückt handelt es sich bei diesem System um eine miniaturisierte Affinitätschromatographie auf einer Gold-Sensoroberfläche. Die Menge eines spezifisch gebundenen Liganden kann in Form eines Resonanzsignales bildlich dargestellt werden (Chaiken, I.; Rose, S.; Karlsson, R. (1992) Anal. Biochem. 201; 197 bis 201; Karlsson, R.; Altschuh, D.; van Regenmortel, M.H.V. (1992) Measurement of antibody affinity, in: Structure of Antigens; CRC Press (van Regenmortel, ed); Boca Raton, Fl.; 127 bis 148).

### 1. Direktes Screening auf immobilisiertem Laminin P1

Laminin P1 wurde gemäß den Instruktionen des User-Manuals bei einer Konzentration von 200 µg/ml in 10mM Na-Acetat pH = 4,0 auf dem Sensorchip immobilisiert. Zur Regeneration der Lam-P1 Affinitätsmatrix kann ein Doppelpuls à 4 µl 100mM HCl durchgeführt werden. Die Laminin P1 Schicht ist äußerst stabil und kann ca. 2 Monate (> 300 Einzelanalysen) ohne Qualitätsverlust verwendet werden.
Für das Screening nach potenten Antikörpern wurden 4 bis 25 *µ*l Kulturüberstand aus den einzelnen Zellkolonien direkt über die Affinitätsmatrix geleitet, nach 1 bis 5 Minuten ist die Bindung (relative Stärke) am RU und an der Konstanz des Signals zu erkennen. Dies erlaubt eine schnelle und zugleich aussagekräftige Auswahl interessanter Klone. Aufeinanderfolgende Injektionen verschiedener Kulturüberstände ohne zwischengeschaltete Regeneration ermöglichen das Screening von Antikörpern die gleichzeitig an unterschiedliche Epitope des Antigens binden können.

### 2. Subklassenbestimmung

Hybridomaüberstände wurden wie oben beschrieben über die Lam-P1 Schicht des BIAcore^{®} Chips geleitet. Nach dem Binden des Antikörpers (Durchfluß der Probe) wurden dann sequentiell je 4 µl von subklassen-spezifischen anti Maus Antikörpern injiziert. Eine Signalerhöhung ist wiederum der Ausdruck für eine gegenseitige Bindung. Damit ist eine eindeutige Zuordnung der Subklasse innerhalb von 5 Minuten möglich.

### 3. Konzentrationsbestimmung

Für eine selektive und reversible Bindung von Mausantikörpern kann auf dem Sensorchip des BIAcore^{®} ein spezieller Antikörper (Rabbit anti mouse Fc-specific; RAM-Fc) mit Hilfe einer Standardmethode immobilisiert werden. Da das Signal des Meßsystems direkt von der Masse des gebundenen Liganden abhängt, ist es möglich Konzentrationsbestimmungen durchzuführen, v.a. wenn das Standardmaterial und die zu analysierende Probe gleiche Molekulargewichte haben. Die Konzentrationsbestimmung eines spezifischen Proteins ist sogar in komplexen Mischungen möglich, da wie im vorliegenden Beispiel durch die Spezifität der Bindung nur die Masse des gewünschten Liganden am Sensorchip festgehalten wird.
Eine Standardkurve zur Bestimmung der in den Kulturüberständen vorliegenden Antikörpermenge (= Syntheseleistung des Klons) wurde mit Hilfe eines im Labor vorliegenden gut charakterisierten monoklonalen Antikörpers (MAK 238) erzeugt. Die aus Doppelbestimmungen gemittelten Signale von unbekannten Proben (Kulturüberständen) können über die Standardkurve in Konzentrationen (µg/ml monoklonalen Antikörper) umgerechnet werden. Das BIAcore System bedient sich bei der Abarbeitung derart komplexer und zeitaufwendiger Serienmessungen eines Randomisierungsprogrammes, so daß solche Fehlerquellen negiert werden, die sich durch die Beanspruchung der Affinitätsmatrix oder durch die unterschiedlichen Standzeiten ergeben könnten.

### 4. Screening nach Bindungseigenschaften zum Humanlaminin

Aufgrund der nur geringen Anreicherung des Humanlaminins in der Präparation (Laminin Charge I) konnte keine direkte Immobilisierung (wie z.B. beim Lam-P1) durchgeführt werden. Es mußte also eine Affinitätsmatrix konstruiert werden, die in der Lage war das Laminin aus der heterogenen Lösung zu fischen.
Auf dem immobilisierten RAM-Fc (s.o.) wurden spezifische Antikörper aus den Kulturüberständen gebunden und somit (nicht-kovalente) Affinitätsmatrices für Humanlaminin erzeugt. Über diese spezifischen Schichten konnte die Laminin-Probe geleitet werden. Im Falle einer Erkennung war ein erneuter Anstieg des Signals zu beobachten.

### 5. Bestimmung von Bindungskonstanten

Mit dem BIAcore^{®} System ist es möglich die Bindung von Liganden quantitativ zu bestimmen (Chaiken, I.; Rosé, S.; Karlsson, R. (1992) Anal. Biochem. 201; 197 bis 201; Karlsson, R.; Altschuh, D.; van Regenmortel, M.H.V. (1992) Measurement of antibody affinity, in: Structure of Antigens; CRC Press (van Regenmortel, ed); Boca Raton, Fl.; 127 bis 148), da die Assoziationsphase, das Einstellen des Bindungsgleichgewichts und die Dissoziationsphase als zeitlich getrennte Prozesse darstellbar sind. Die Software des Gerätes ermöglicht eine relativ einfache Überführung der Meßdaten in die entsprechenden Tabellenkalkulationen.
Für die Bestimmung der Bindungskonstanten muß (z.B.) ein spezifischer Antikörper (eine vorherige Reinigung ist nicht unbedingt erforderlich!) in mehreren Verdünnungen über die Affinitätsmatrix geleitet werden. Während der Assoziationsphase ermittelt das Programm in benutzerdefinierten Intervallen die RU-Werte und die aktuelle Steigung der Kurve, sodaß eine Auftragung slope/R möglich ist. Die Assoziationskonstante kₐₛₛ kann bestimmt werden, wenn die Steigungen dieser Funktion für alle analysierten Konzentrationen gegen die vorliegenden aktuellen Konzentrationen (in nM) in Beziehung gesetzt werden. Bei der höchsten Antikörperkonzentration wird die Dissoziationsphase im Experiment extrem verlängert. Aus der Auftragung InR1/Rn gegen die Zeit kann die Dissoziationskonstante k_{diss} ermittelt werden. Die Gleichgewichtskonstante KD ergibt sich aus der Formel kₐₛₛ/k_{diss}.

**Tabelle 1:**

| | Subklasse | Bindung Humanlminin (BlAcore) | Bindung Serumlaminin Peak 1 (hochmolek.) | Bindung Serumlaminin Peak 2 (niedermolek.) |
|---|---|---|---|---|
| A27/2/1 | IgG 2a | 534 RU | 19,5 µg/ml ⁽¹ | 9,6 µg/ml ⁽¹ |
| A9/2/1 | IgG 2a | 601 RU | 20,5 µg/ml ⁽² | 2,4 µg/ml ⁽² |
| A33/2/20 | IgG 1 | 154 RU | 12,3 µg/ml ⁽² | 1,9 µg/ml ⁽² |

| | | | | |
|---|---|---|---|---|
| 1) IRMA-Verfahren mit RIA-Ak (= polyklonales Kaninchen lgG; Grundlage des RIA-gnost^{®}) als Schichtantikörper und jodiertem A27/2/1. 2) IRMA-Verfahren mit A27/2/1 als Schichtantikörper und jodierten A9/2/1, A33/2/20. | | | | |

**Tabelle 2:**

| Physikalische Eigenschaften - Bindungskonstanten | | | |
|---|---|---|---|
| A) Bindungskonstanten auf einer Lam P1 Affinitätsmatrix | | | |
| | A27/2/1 | A9/2/1 | A33/2/20 |
| kₐₛₛ (1/Ms) | 2,972x10⁵ | 2,303x 10⁵ | 1,72x10⁵ |
| k_{diss} 1/s) | 6,71x10⁻⁶ | 8,699x10⁻⁶ | 1,28x10⁻⁶ |
| KD (1/M) | 4,43x10¹⁰ | 2,65x10¹⁰ | 1,35x10¹⁰ |

| B) Bindungskonstanten auf einer Human-Laminin Affinitätsmatrix | | | |
|---|---|---|---|
| | A27/2/1 | A9/2/1 | A33/2/20 |
| kₐₛₛ (1/Ms) | 1,25x10⁵ | 4,54x10⁵ | 4,74x10⁴ |
| k_{diss} (1/s) | in 30 min | nicht | meßbar l |
| KD (1/M) | - | - | - |

**Tabelle 3:**

| Physikalische Eigenschaften - Bindungskonstanten | | | |
|---|---|---|---|
| Bindungskonstanten auf einer MAK A27/2/1 Affinitätsmatrix | | | |
| | Lam P1 | Laminin* | Laminin** |
| kₐₛₛ (1/Ms) | 8,64x10³ | 1,14x10⁵ | 1,20x10⁵ |
| k_{diss} (1/s) | 2,24x10⁻⁴ | 1,52x10⁻⁴ | 1,17x10⁻⁴ |
| KD (1/M) | 3,85x10⁷ | 7,49x10⁸ | 1,03x10⁹ |

| | | | |
|---|---|---|---|
| * Humanlaminin gereinigt wie unter Methoden beschrieben ** Humanlaminin gereinigt über Affinitätschromatographie an monoklonalen Antikörpercoctail | | | |

Aus den drei Tabellen geht hervor, daß die drei monoklonalen Antikörper sehr gute Bindungseigenschaften besitzen, v.a. zeichnen sich die Antikörper durch eine sehr feste Bindung an das immobilisierte Antigen aus. Im Falle der Lamininschicht ist z.B. während des Analysenzeitraumes (30 min) bei keinem der Antikörper eine Dissoziation feststellbar. Wird allerdings gelöstes Antigen z.B. über eine A27/2/1 Schicht geleitet, so erfolgt die Assoziation mit unverändert schneller Kinetik (kass1,2x10⁵ /Ms). Es schließt sich aber dann eine deutliche Dissoziationsphase an, sodaß eine Gleichgewichtskonstante von etwa 1x10⁹ für die Bindung des Laminins bestimmt werden kann. Interessanterweise unterscheiden sich die Bindungskonstanten zum Lam P1 um den Faktor 1000 in Abhängigkeit davon, ob das LamP1 immobilisiert (lokal hohe Konzentration) vorliegt oder ob es aus der Lösung gebunden werden muß. Dieser Effekt könnte die Grundlage für die exclusive Erkennung des hochmolekularen Peaks (intaktes Serumlaminin) im Humanserum sein (siehe unten).

**Tabelle 4**

| Screeningstrategie für mk. anti LamP1/Laminin Antikörper | | |
|---|---|---|
| Screeningstufen | Testmethode/Auswahlkriterium | Auswahl |
| Immunisierung | Titerbestimmung | |
| | | |
| Fusionierung → Hybridomas | Screening mit ELISA auf Lam P1 AUSWAHLKRITERIEN: | 150 pos. Klone |
| | Erkennung von Lam-P1 im ELISA (Höhe der OD) | |
| | Wachstum der Klone in der Zellkultur | |
| 1. Selektion | | 56 pos. Klone |
| | | |
| Kultur in 24 well Kulturschalen | Screening mit ELISA auf Lam-P1 und auf laminin-angereichertem Extrakt aus Humanplazenta | |
| | AUSWAHLKRITERIEN: | |
| | starke Bindung auf Lam-P1 und/oder positive Reaktion auf Laminin | |
| 2. Selektion | | 18 pos. Klone |
| | | |
| Erste biochemische Charaterisierung | Subklassenbestimmung mit BIAcore (Ausschluß von IgM) Screening mit SDS-Gelelektrophorese, Western-Blot, Immunfärbung | |
| | AUSWAHLKRITERIEN: | |
| | Reaktion mit unreduziertem und/oder reduziertem Laminin | |
| Vereinzelung der positiven Klone (pro Klon etwa 10-20 Subkolonien) | | |
| | | |
| Zweite biochem. Charakterisierung | Subklassenbestimmung mit BIAcore (Ausschluß von IgM) Screening mit SDS-Gelelktrophorese, Western-Blot, Immunfärbung | |
| | AUSWAHLKRITERIEN: | |
| | Reaktion mit unreduziertem und/oder reduziertem Laminin | |
| 3. Selektion | | 11 pos. Klone |
| | | |
| Charakterisierung mit BlAcore | Überprüfung der Lam-P1-Bindung (rel. Stärke) | |
| | Überprüfung der Laminin-Bindung (rel. Stärke) | |
| | Subklassenbestimmung (Sorteneinheit versch. Klone) | |
| | qualitative Bewertung der Assoziations- und Dissoziationskinetiken | |
| | AUSWAHLKRITERIEN: | |
| | stärkste Bindung auf Lam-P1 Bindung von Laminin aus Plazentaextrakt | |
| | Ausschluß von IgM (in 2 Fällen) Klone mit der höchsten Syntheseleistung | |
| | Simulatane Bindung mit anderen monoclonalen Antikörpern am Antigen | |
| 4. Selektion | | 5 pos. Klone |
| | | |
| Reaktion mit Serum | Nachweis der hochmolekularen Serumform (RIA) | |
| | Hohe Affinität zum "Serumantigen"? (RIA) | |
| | Erste Stufen der Assayentwicklung (Tabelle 5) | |
| 5. Selektion | | 3 pos. Klone . |
| | | |
| Dritte biochem. Charakterisierung | Elektrophorese, Western-Blot, Immunfärbung | |
| | Bestimmung von Bindungskonstanten mit BIAcore | |
| | Ausarbeitung der Reiniungsbedingungen für monoklonale Antikörper | |

**Tabelle 5**

| Erste Stufen der Assayentwicklung | | |
|---|---|---|
| Erkennung des Serumantigens | | Beschichtungsversuche |
| mit Hilfe einer coated tube Verfahrens auf der Grundlage des polyclonalen Antiserums aus RIA-gnost® LamP1. Die 5 ausgewählten monoklonalen Antikörper (4. Selektion) wurden als jodierte Zweitantikörper eingesetzt. | Produktion und Reinigung der monoclonalen Antikörper im 1-5mg Maßstab | verschiedene RIA-Röhrchen wurden mit den ausgesuchten Antikörpern (4. Selektion) beschichtet, als Sonde für die Oberflächenbindung wurde jodiertes LamP1 verwendet. Optimierung des Beschichtungspuffers und der Blockierungsbedingungen |
| Testantigene: gereinigtes Serumlaminin (high Mw- und low Mw-Formen); LamP1 Standard aus RIA-gnost^{®} LamP1; Normalseren | Produktion von A27/2/1 und A9/2/1 (100mg Maßstab) | → bester Kanidat als Schichtantikörper = A27/2/1 B2 |
| Auswahlkriterien für coated tube Assays: Erkennung von LamP1-Standard, Serum Laminin (high Mw)deutliclie Reaktion mit Normalserum Verdünnungsechtheit des Tests | | Optimierung der Inkubationszeiten, . Inkubationsintervalle, Puffer, Serumvolumina für 3 coated tube Varianten |

### Fortsetzung der Testbedingungen für drei coated tube Verfahren

| | |
|---|---|
| Schichtantikörper = | A27/2/1 B2 |
| Zweitantikörper | = A9/2/1 B2 |
| | = A33/2/20 |
| | = polyclonales anti LamP1 IgG |

### TESTMESSUNGEN mit den drei coated tube Verfahren

Bestimmung der Antigenverteilung in Normalseren
Bestimmung der Normalgehalte
Test auf cross-Reaktivitäten
Anwendung auf unterschiedliche Indikationen

**Tabelle 6**

| | | | | | | | | | | | | |
|---|---|---|---|---|---|---|---|---|---|---|---|---|
| Charakterisierung der monoklonalen anti Laminin P1 Antikörper | | | | | | | | | | | | |
| | | | | | | | | | | | | |
| Die Konzentrationsbestimmung und Subklassenbestimmung erfolgte mit BlAcore. | | | | | | | | | | | | |
| Die Werte in der Spalte "ELISA" sind als OD bei 405 nm angegeben. | | | | | | | | | | | | |
| In den Spalten "Blot" bedeutet -SH unreduziert, +SH reduziert, + + + sehr starke Reaktion, + + starke Reaktion, + deutliche Reaktion, - keine Reaktion. Für die Bindungsstudien im BIAcore wurden je zwei Chargen Laminin P1 (Ch. 1, Ch. 2) und Laminin (Ch. 1, Ch. II) herangezogen. | | | | | | | | | | | | |
| In der Spalte "Paare" sind die Antikörper bzw. Klone angegeben, die mit dem in Spalte 1 spezifizierten Antikörper gleichzeitig am Laminin P1 binden können. | | | | | | | | | | | | |

| **Charakterisierung der monoklonalen anti Laminin P1 Antikörper** | | | | | | | | | | | | |
|---|---|---|---|---|---|---|---|---|---|---|---|---|
| Antikörper | Konz.µg/mg | Subtyp | ELISA | | Blot | | BIAcore | | | | | Paare |
| | | | Lam P1 | Laminin (S) | Laminin - SH | Laminin + SH | CH.1.25µl | Lam P1 CH.2.4µl | CH.2.25µl | Lamin CH.1 | (H) CH.2 | |
| A 27/1/1 | 10 | | 1.57 | 1.061 | +++ | - | | 191 RU | 584 RU | | | A9/1/7; A24/1/13; A25/1/2 |
| A 27/1/2 | 20.9 | | 1.224 | 0.942 | | | | | 638 RU | | | |
| A 27/1/7 | 10.1 (5.2) | IgG2a | 1.488 | 1.058 | | | | 231 RU | 613 RU | | | A50/1/4 |
| A 27/1/8 | 12.8 | IgG2a | 1.503 | 1.038 | +++ | + + | | | 650 Ru | | 291 | |
| A 9/1/7 | 23.6 | IgG2a | 1.544 | 1.147 | +++ | schwach | | 514 RU | 1050 RU | | 332 | A27/1/7; A25/1/2; A50/1/4 |
| A 9/1/12 | 12.1 | IgG2a | 1.289 | 0.906 | | | | 418 RU | 774 | | 300 | |
| A 9/1/4 | 11.3 | | 1.556 | 1.046 | | | | | 794 | | 309 | |
| A 9/1/9 | 31.0 | | 1.559 | 1.141 | | | | | (1:3)661 | | 323 | |
| A 24/1/13 | 10.8 | IgG2a | 1.319 | 1.628 | +++ | schwach | 833 RU | 345 RU | 737 RU | + | | A33/1; A27/1/7; A25/1/2 |
| A 24/1/5 | 21.3 | | 1.266 | 0.922 | | | | | | | 280 | A50/1/5; A33/1/1; A50/1/4 |
| A 24/1/23 | 21.5 | | 1.782 | 0.854 | | | | | | | 276 | |
| A 25/1/2 | | IgG2a:IgG1 | 2.079 | 2.125 | ++(1:500) | - | 410 RU | 443 RU | 331 RU (1:10) | + | - | A24/1/6; A27/1/1; A50/1/4 |
| A 35/1/2 | 12.9 | IgG1 | 0.816 | 0.01 | schwach | + | 148 RU | 48 RU | 129 RU | | | |
| A 33/1/3 | 14.1 | IgG1:IgM | 1.004 | 0.122 | | | | 143 RU | 404 RU | | | |
| A 33/1/1 | 7 | IgM | 1.214 | 0.02 | schwach | - | | 161 RU | | | | |
| A 6/1/7 | 7 | IgM | 1.386 | 1.517 | + + | schwach | 622 RU | 272 RU | | + | | |
| A 6/1/8 | 7 | lgM:lgG1 | 1.632 | 1.46 | | | 640 RU | 275 RU | 495 RU | | | |
| A 16/1/1 | 6.0 | lgG1 | 0.561 | 0.628 | schwach | - | | 26 RU | 77 RU | | | |
| A 16/1/6 | 8.6 | lgG1 | 0.76 | 0.592 | | | | 35 RU | 103 RU | | | |
| A 41/1/7 | 9.1 | lpG1 | 0.327 | 0.416 | schwach | - | | 6 RU | | | | |
| A 50/1/4 | 16.3 | lgG1 | 1.289 | - | | | | 681 RU | 947 RU | | - | A9/1/7; A24/1/13; A27/1/8 |
| A 50/1/5 | 16.6 | lgG1 | 1.406 | - | schwach | | 261 RU | 157 RU | 185 RU | | - | A24/1/13; A28/1/1 |
| A 48/1/5 | 18.5 | lgG1 | 0.33 | - | | | | 241 RU | 372 RU | | | |
| A 46/1/12 | 38.5 | lgG1 | 0.319 | - | | | | 380 RU | 576 RU | | - | A9/1/7; A24/2/2; A27/1/8 |
| | | | | | | | | | | | | (A25/1/2) |

**Tabelle 7**

| Endgültige Entscheidungskriterien zur Auswahl von Klonen für eine Testentwicklung | | | | | | | | | | | |
|---|---|---|---|---|---|---|---|---|---|---|---|
| | Sandwich mit Poly K4 (1/93) | | | | | | | | | | |
| Beschichtung | Verd/µg/ml | | Standard | | cpm | %Bind | Lam Chl | SerumP1 | SerumP2 | Human-serum | cpm |
| | | | ng/ml | | | | ug/ml | ug/ml | ug/ml | | |
| | | | | | | | | | | | |
| | | | | | | | | | | 200 ul | |
| 28/1/1 v. 7.6.91 | 1:20 | 49 | S0 | | 70 | | 10,6 | 14 | 1,7 | 300/90 | 699 |
| | | | S1 | 35,6 | 5632 | | | | | Testserum | 1217 |
| | | | S4 | 284 | 29721 | | | | | 52/89 | 843 |
| | | | S7 | 2400 | 79393 | 51 | | | | | |
| | | | | | | | | | | | |
| | | | | | | | | | | 200 ul | |
| 24/2/2 (075) | 1:10 | 12,6 | S0 | | 116 | | 5,7 | 9,6 | 1,6 | 300/90 | 145 |
| | | | S1 | 35,6 | 4073 | 3 | | | | Testserum | 489 |
| | | | S4 | 284 | 22989 | 16 | | | | 52/89 | 172 |
| | | | S7 | 2400 | 58777 | 42 | | | | | |
| | | | | | | | | | | | |
| | | | | | | | | | | 200 ul | |
| 27/2/1 (071) | 1:10 | 190 | S0 | | 167 | | 19,8 | 9,9 | 1,6 | 300/90 | 11621 |
| | | | S1 | 35,6 | 5155 | | | | | Testserum | 19554 |
| | | | S4 | 284 | 30580 | | | | | 52/89 | 15602 |
| | | | S7 | 2400 | 71761 | | 46 | | | | |
| | 1:30 | 65 | S0 | | 193 | | | | | 300/90 (100ul) | 11117 |
| | | | S1 | 35,6 | 5623 | | | | | | |
| | | | S4 | 284 | 29596 | | | | | | |
| | | | S7 | 2400 | 66547 | 47 | | | | | |
| | | | | | | | | | | 200 ul | |
| 27/2/1 (074) | 1:10 | 26 | S0 | | 66 | | 19,3 | 9,4 | 1,7 | 300/90 | 9234 |
| | | | S1 | 35,6 | 4931 | 3 | | | | Testserum | 13258 |
| | | | S4 | 284 | 25524 | 18 | | | | 52/89 | 13843 |
| | | | S7 | 2400 | 63453 | 45 | | | | | |
| | | | | | | | | | | | |
| | | | | | | | | | | 200 ul | |
| 9/2/1 (072) | 1:10 | 10 | S0 | | 91 | | 12,7 | 11 | 1,3 | 300/90 | 1480 |
| | | | S1 | 35,6 | 5198 | 3,6 | | | | | 2995 |
| | | | S4 | 284 | 28286 | 20 | | | | | 1999 |
| | | | S7 | 2400 | 63388 | 45 | | | | | |

| | | | | | | | | | | | |
|---|---|---|---|---|---|---|---|---|---|---|---|
| "Standard cpm bzw. % Bindung" Vergleich der RIAgnost Standard Erkennung "Lam Chl" Bindung an Laminin, das wie unter Methoden beschrieben gereinigt wurde "Serum P1, Serum P2" hochmolekulares bzw. niedermolekulares Serumantigen; gereinigt aus ≈ 20 ml humanem Normalserum "Humanserum" Bindung des Antigens in humanen Testseren. Die Klone A28/1/1 und A24/2/2 sind aufgrund der ungenügenden Reaktion ungeeignet. | | | | | | | | | | | |

**Tabelle 8**

| Kreuzreaktionen | | | | | | | |
|---|---|---|---|---|---|---|---|
| Tracer | | A9/2/1B2 | | | A33/2/20 | | |
| Antigen | Testkonzentration | counts | %Binding | Konzentration | counts | %Binding | Konzentration |
| | E/ml (µg/ml) | | | berechnet | | | berechnet |
| LamP1 | 0 | 289 | 0,18 | | 102 | 0,09 | |
| Standard | 0,17 (0,037) | 4140 | 2,6 | | 1606 | 1,41 | |
| | 1,43 (0,31) | 32050 | 20,2 | | 13066 | 11,5 | |
| | 12 (2,64) | 142523 | 89,9 | | 51264 | 45 | |
| Kollagen 1 | 5 | 607 | 0,38 | | 807 | 0,71 | |
| (Chemicon) | 1 | 340 | 0,21 | | 287 | 0,25 | |
| Kollagen III | 5 | 331 | 0,21 | | 138 | 0,12 | |
| (Chemicon) | 1 | 316 | 0,2 | | 114 | 0,1 | |
| Kollagen IV | 5 | 527 | 0,33 | | 120 | 0,11 | |
| (Chemicon) | 1 | 333 | 0,21 | | 114 | 0,1 | |
| Kollagen V | 5 | 614 | 0,39 | | 241 | 0,21 | |
| (Chemicon) | 1 | 455 | 0.29 | | 232 | 0.2 | |
| Kollagen VI | 6.6 | 378 | 0,24 | | 262 | 0.23 | |
| (Chemicon) | 1,32 | 533 | 0.34 | | 161 | 0,14 | |
| Fibronectin | 5 | 413 | 0.26 | | 141 | 0,12 | |
| (Chemicon) | 1 | 463 | 0,29 | | 148 | 0,13 | |
| Fibrinogen | 5 | 367 | 0.23 | | 171 | 0,15 | |
| (sigma) | 1 | 279 | 0,18 | | 151 | 0,13 | |
| Kol.VI a3Nt | 3,3 | 349 | 0,22 | | 148 | 0,13 | |
| Dr. Timpl | 0,66 | 381 | 0,24 | | 130 | 0,11 | |
| NC 1 | 5 | 330 | 0,21 | | 122 | 0,11 | |
| Eigenisolat | 1 | 407 | 0.26 | | 174 | 0,15 | |
| 7S (DE10) | 5 | 303 | 0,19 | | 159 | 0,14 | |
| Eigenisolat | 1 | 311 | 0,2 | | 132 | 0,12 | |
| 7S (DE6) | 5 | 271 | 0,17 | | 160 | 0,14 | |
| Eigenisolat | 1 | 336 | 0,21 | | 230 | 0,2 | |
| EHS-LN | 7 | 279 | 0,18 | | 186 | 0,16 | |
| Dr. Timpl | 1,4 | 306 | 0,19 | | 250 | 0,22 | |
| EHS-LamP1 | 6,8 | 466 | 0,29 | | 270 | 0,24 | |
| Dr. Timpl | 1,36 | 381 | 0,24 | | 282 | 0,25 | |
| Laminin (Chemicon) Merosin (Chemicon) | 6,73 | 18210 | 11,5 | 0,176 *µ*g/ml | 4009 | 3,52 | 0,095 *µ*g/ml |
| | 1,35 | 4290 | 2,7 | 0,039 *µ*g/ml | 974 | 0.86 | |
| | 5 | 127867 | 80,7 | 2,05 *µ*g/ml | 25468 | 22,3 | 0,68 *µ*g/ml |
| | 1 | 35812 | 22,6 | 0,36 *µ*g/ml | 5632 | 4,9 | 0,13 *µ*g/ml |

**Tabelle 9**

| Normalwerte: | | | |
|---|---|---|---|
| | n | Mittelwert E/ml | Median E/ml |
| RIA-gnost^{®} Lam-P1 | 126 | 1,32 ± 0,18 | 1,31 |
| A27/2/1-A9/2/1 | 23 | 0,864 ± 0,212 | 0,849 |
| A27/2/1-A33/2/20 | 24 | 0,073 ± 0,038 | 0,065 |

**Tabelle 10:**

| Primär Biliäre Zirrhose | | | | | |
|---|---|---|---|---|---|
| | n | Mittelwert E/ml | Median E/ml | Erhöhung vgl.mit Normalwert | Korrelation zu RIA-gnost^{®} r² |
| RIA-gnost^{®} Lam-P1 | 42 | 2,28 ± 0,57 | 2,41 | 1,72 | |
| A27/2/1-A9/2/1 | 42 | 1,98 ± 0,96 | 2,03 | 2,29 | 0,49 |
| A27/2/1-A33/2/20 | 42 | 0,45 ± 0,4 | 0,34 | 6,16 | 0,28 |

**Tabelle 11**

| Alkoholisch toxische Leberzirrhose | | | | | |
|---|---|---|---|---|---|
| | n | Mittelwert E/ml | Median E/ml | Erhöhung vgl.mit Normalwert | Korrelation zu RIA-gnost^{®} r² |
| RIA-gnost^{®} Lam-P1 | 58 | 3,14 ± 0,98 | 3,15 | 2,38 | |
| A27/2/1-A9/2/1 | 58 | 3,71 ± 2,08 | 3,19 | 4,29 | 0,69 |
| A27/2/1-A33/2/20 | 58 | 0,9 ± 0,59 | 0,69 | 12,3 | 0,21 |

**Tabelle 12**

| Chronisch aktive Hepatitis | | | | | |
|---|---|---|---|---|---|
| | n | Mittelwert E/ml | Median E/ml | Erhöhung vgl.mit Normalwert | Korrelation zu RIA-gnost^{®} r² |
| RIA-gnost^{®} Lam-P1 | 26 | 2,20 ± 0,58 | 2,45 | 1,7 | |
| A27/2/1-A9/2/1 | 26 | 2,35 ± 1,09 | 2,16 | 2,7 | 0,26 |
| A27/2/1-A33/2/20 | 22 | 0,53 ± 0,45 | 0,38 | 7,3 | 0,05 |

**Tabelle 13**

| Dekompensierte Leberzirrhose | | | | | |
|---|---|---|---|---|---|
| | n | Mittelwert E/ml | Median E/ml | Erhöhung vgl.mit Normalwert | Korrelation zu RIA-gnost® r² |
| RIA-gnost^{®} Lam-P1 | 23 | 2,71 ± 1,29 | 2,26 | 2,05 | |
| A27/2/1-A9/2/1 | 19 | 3.05 ± 1,77 | 2,84 | 3,53 | 0,93 |
| A27/2/1 -A33/2/20 | 19 | 0,664 ± 0,556 | 0,38 | 9,14 | 0,41 |

**Tabelle 14**

| Leberzirrhose unbestimmten Ursprungs | | | | | |
|---|---|---|---|---|---|
| | n | Mittelwert E/ml | Median E/ml | Erhöhung vgl.mit Normalwert | Korrelation zu RIA-gnost^{®} r² |
| RIA-gnost^{®} Lam-P1 | 15 | 3,25 ± 1,03 | 3,18 | 2,46 | |
| A27/2/1-A9/2/1 | 14 | 4,52 ± 1,99 | 4,48 | 5,23 | 0,43 |
| A27/2/1-A33/2/20 | 14 | 1.09 ± 0,564 | 1,14 | 14,9 | 0,8 |

**Tabelle 15**

| Posthepatische Leberzirrhose | | | | | |
|---|---|---|---|---|---|
| | n | Mittelwert E/ml | Median E/ml | Erhöhung vgl.mit Normalwert | Korrelation zu RIA-gnost^{®} r² |
| RIA-gnost^{®} Lam-P1 | 26 | 2,96 ± 1.03 | 2,93 | 2,24 | |
| A2712/1-A9/2/1 | 25 | 2,82 ± 1,3 | 2,29 | 3,26 | 0,35 |
| A2712/1-A3312120 | 25 | 0,583 ± 0,452 | 0,45 | 7,99 | 0,28 |

**Tabelle 16**

| Krebserkrankungen | | | | | | |
|---|---|---|---|---|---|---|
| A. Serum eines Patienten mit hepatozellulärem Karzinom | | | | | | |
| | | RIA-gnost^{®} Lam-P1 | | A27/2/1-A9/2/1 | | A27/2/1-A33/2/20 |
| Laminin E/ml | | 3,4 | | 3,6 | | 0,45 |
| Erhöhung | | 2,6 | | 4,2 | | 6,2 |

| B. Patienten mit Lungenkrebs | | | | | | |
|---|---|---|---|---|---|---|
| Patient | RIA-gnost^{®} Lam-P1 | | A27/2/1-A9/2/1 | | A27/2/1-A33/2/20 | |
| | E/ml | | E/ml | | E/ml | |
| MC | 3,01 | | 1,81 | | 0,183 | |
| JV | 2,7 | | 1,25 | | 0,054 | |
| EU | 2,8 | | 2.4 | | 0,200 | |

**Tabelle 17**

| Diabetes | | | | | |
|---|---|---|---|---|---|
| | n | Mittelwert E/ml | Median E/ml | Abweichung vgl.mit Normalwert | Korrelation zu RIA-gnost^{®} r² |
| RIA-gnost^{®} Lam-P1 | 78 | 1,70 ± 0,30 | 1,66 | 1,3 | |
| A27/2/1-A9/2/1 | 80 | 0,73 ± 0,37 | 0,58 | 0,84 | 0,61 |
| A27/2/1-A33/2/20 | 80 | 0,057 ± 0,064 | 0,039 | 0,53 | 0,05 |

Untersucht wurden die Seren von 80 stationär zugewiesenen Diabetikern (32% Typl, 68% TypII) mit einer Diabetesdauer von 11-14 Jahren (Stracke, H.; Wiek, K.; Günzler, V.; Federlin, K. (1993) Die Medizinische Welt 44; 383 bis 385), bei denen Cheiropathie (36%), Retinopathie (48%), Neuropathie (66%) und Nephropathie (39%) diagnostiziert wurde.

## Patentansprüche

1. Monoclonaler Antikörper mit Spezifität zu Proteinen aus der Familie der Laminine und dem durch Pepsinverdau aus Humanplazenta herstellbaren Laminin P1 Fragment, **dadurch gekennzeichnet, daß** der monoklonale Antikörper bevorzugt an den nativ gefalteten Strukturen der Laminin P1 Domäne des Laminins bindet, die Affinität des Antikörpers zum intakten, nativen Laminin ungefähr gleich der Affinität des Antikörpers zum Laminin P1 Fragment ist und der Antikörper die Eigenschaften eines monoklonalen Antikörpers aufweist, der von der Hybridoma-Zellinie DSM ACC2181, DSM ACC2180 oder DSM ACC2182 produziert wird.

2. Monoclonaler Antikörper nach Anspruch 1, **dadurch gekennzeichnet, daß** der Antikörper durch ein Hybridom gebildet wird, das durch Fusion von Zellen aus einer Myelom-Zellinie und Lymphozyten von einem zuvor mit Laminin P1 immunisierten nicht-humanen Wirbeltier entstanden und anschließend danach ausgewählt worden ist, dass der gebildete Antikörper neben einer guten Bindungseigenschaft zum gereinigten Humanlaminin aus Plazenta auch eine gute Reaktion mit der hochmolekularen Form des aus Humanserum gewonnenen Laminins zeigt.

3. Monoclonaler Antikörper nach Anspruch 1 oder 2, **gekennzeichnet durch** die Fähigkeit, paarweise mit einem weiteren Antikörper gemäß Anspruch 1 oder 2 an das Antigen zu binden.

4. Monoclonaler Antikörper nach einem der Ansprüche 1 bis 3, **dadurch gekennzeichnet, daß** er der Subklasse lgG 2a zugeordnet wird.

5. Monoclonaler Antikörper nach einem der Ansprüche 1 bis 3, **dadurch gekennzeichnet, daß** er der Subklasse lgG 1 zugeordnet wird.

6. Hybridoma-Zellinie, die einen Antikörper gemäß einem der Ansprüche 1 bis 3 produziert, herstellbar durch Fusion von Zellen aus einer Myelom-Zellinie und Lymphozyten von einem zuvor mit Laminin P1 immunisierten nicht humanen Wirbeltier und nachfolgender Auswahl der Hybride danach, daß der von dem Hybrid produzierte Antikörper neben einer guten Bindungseigenschaft zum gereinigten Humanlaminin aus Plazenta auch eine gute Reaktion mit der hochmolekularen Form des aus Humanserum gewonnenen Laminins zeigt.

7. Hybridoma-Zellinie nach Anspruch 6, **dadurch gekennzeichnet, dass** die Lymphozyten aus mit Laminin P1 immunisierten Mäusen des Balb/c-Stammes entnommen sind und die Myelom-Zellinie die Maus-Myelom-Zellinie P3X63AG8.653 ist.

8. Die Hybridoma-Zellinie DSM ACC2181.

9. Die Hybridoma-Zellinie DSM ACC2180.

10. Die Hybridoma-Zellinie DSM ACC2182.

11. Verfahren zur Herstellung eines monoklonalen Antikörpers gemäß einem der Ansprüche 1 bis 5, **dadurch gekennzeichnet, daß**
a) nicht-humanen Wirbeltiere mit dem durch Pepsinverdau von humaner Plazenta herstellbaren Laminin P1 Fragment immunisiert werden,
b) Lymphozyten der immunisierten Wirbeltiere gewonnen und mit Myelom-Zellen fusioniert werden,
c) die Hybride hinsichtlich des Vorliegens eines Antiköpers mit den in den Ansprüchen 1 bis 3 angegebenen Eigenschaften ausgewählt und geklont werden und
d) der Antikörper aus diesen Klonen gewonnen wird.

12. Verfahren nach Anspruch 11, **dadurch gekennzeichnet, daß** zur Ausführung des Verfahrensschritts d) in Anspruch 11 die Hybridoma-Zellinien DSM ACC2181, DSM ACC2180 oder DSM ACC2182 eingesetzt werden.

13. Verfahren zur immunologischen Bestimmung von nativem Laminin unter Verwendung eines adhäsiv oder kovalent an ein Trägermaterial fixierten Schichtantikörpers und eines markierten Zweitantikörpers, der das an den Schichtantikörper gebundene Antigen erkennt, **dadurch gekennzeichnet, daß** der Schichtantikörper ein monoclonaler Antikörper gemäß einem der Ansprüche 1 bis 5 ist.

14. Verfahren zur immunologischen Bestimmung von nativem Laminin unter Verwendung eines adhäsiv oder kovalent an ein Trägermaterial fixierten Schichtantikörpers und eines markierten Zweitantikörpers, der das an den Schichtantikörper gebundene Antigen erkennt, **dadurch gekennzeichnet, daß** der markierte Zweitantikörper ein monoclonaler Antikörper gemäß einem der Ansprüche 1 bis 5 ist.

15. Verfahren nach Anspruch 14, **dadurch gekennzeichnet, daß** der Schichtantikörper ein monoklonaler Antikörper gemäß einem der Ansprüche 1 bis 5 ist.

16. Verfahren nach Anspruche 14 oder 15, **dadurch gekennzeichnet, daß** der Schichtantikörper ein monoklonaler Antikörper ist, der von der Hybridoma-Zellinie DSM ACC2181 produziert wird.

## Claims

1. A monoclonal antibody having specificity for proteins from the family of the laminins and the laminin P1 fragment which can be prepared from human placenta by pepsin digestion, wherein the monoclonal antibody preferably binds to the structures of the laminin P1 domain of laminin which are folded in the native manner, the affinity of the antibody for intact, native laminin is approximately equal to the affinity of the antibody for the laminin P1 fragment and the antibody has the properties of a monoclonal antibody which is produced by the hybridoma cell line DSM ACC2181 DSM ACC2180 or DSM ACC2182.

2. A monoclonal antibody as claimed in claim 1, wherein the antibody is formed by a hybridoma which arose by fusing cells from a myeloma cell line and lymphocytes from a non-human vertebrate, which had previously been immunized with Laminin P1, and was subsequently selected, and wherein the antibody which is formed also exhibits, in addition to good binding affinity for purified human laminin from placenta, a good reaction with the high molecular weight form of the laminin isolated from human serum.

3. A monoclonal antibody as claimed in one of claims 1 to 2, which has the ability to bind to the antigen as a pair together with an additional antibody as claimed in claim 1 or 2.

4. A monoclonal antibody as claimed in one of claims 1 to 3, which is assigned to the IgG 2a subclass.

5. A monoclonal antibody as claimed in one of claims 1 to 3, which is assigned to the IgG 1 subclass.

6. A hybridoma cell line which produces an antibody as claimed in one of claims 1 to 3 and which can be prepared by fusing cells from a myeloma cell line and lymphocytes from a non-human vertebrate, which has previously been immunized with laminin P1, and subsequently selecting the hybrids on the basis of whether the antibody produced by the hybrid also exhibits, in addition to good binding affinity for purified human laminin from placenta, a good reaction with the high molecular weight form of the laminin isolated from human serum.

7. A hybridoma cell line as claimed in claim 6, wherein the lymphocytes are removed from mice of the Balb/c strain which have been immunized with laminin P1 and the myeloma cell line is the mouse myeloma cell line P3X63AG8.653.

8. The hybridoma cell line DSM ACC2181.

9. The hybridoma cell line DSM ACC2180.

10. The hybridoma cell line DSM ACC2182.

11. A process for preparing a monoclonal antibody as claimed in one of claims 1 to 5, wherein
a) non-human vertebrates are immunized with the laminin P1 fragment which can be prepared from human placenta by pepsin digestion,
b) lymphocytes are isolated from the immunized vertebrates and fused with myeloma cells,
c) the hybrids are selected with regard to the presence of an antibody having the properties given in claims 1 to 3 and cloned, and
d) the antibody is isolated from these clones.

12. The process as claimed in claim 11, wherein the hybridoma cell lines DSM ACC2181, DSM ACC2180 or DSM ACC2182 are employed for carrying out process step d) in claim 11.

13. A method for the immunological determination of native laminin using a coating antibody, which is bound adhesively or covalently to a support material, and a labeled second antibody which recognizes the antigen bound to the coating antibody, wherein the coating antibody is a monoclonal antibody as claimed in one of claims 1 to 5.

14. A method for the immunological determination of native laminin using a coating antibody, which is bound adhesively or covalently to a support material, and a labeled second antibody which recognizes the antigen bound to the coating antibody, wherein the labeled second antibody is a monoclonal antibody as claimed in one of claims 1 to 5.

15. The method as claimed in claim 14, wherein the coating antibody is a monoclonal antibody as claimed in one of claims 1 to 5.

16. The method as claimed in claim 14 or 15, wherein the coating antibody is a monoclonal antibody, which is produced by the hybridoma cell line DSM ACC2181.

## Revendications

1. Anticorps monoclonal présentant une spécificité pour des protéines de la famille des laminines et pour le fragment de laminine P1 pouvant être produit par la digestion de pepsines de placenta humain, **caractérisé en ce que** l'anticorps monoclonal se lie de préférence aux structures naturellement pliées des domaines de la laminine P1 de la laminine, l'affinité de l'anticorps pour la laminine naturelle intacte est environ identique à l'affinité de l'anticorps pour le fragment de laminine P1 et l'anticorps présente les propriétés d'un anticorps monoclonal, qui est produit par la lignée cellulaire d'hybridome DSM ACC2181, DSM ACC2180
ou DSM ACC2182.

2. Anticorps monoclonal selon la revendication 1, **caractérisé en ce que** l'anticorps est formé par un hybridome, qui a été formé par fusion de cellules d'une lignée cellulaire de myélome et de lymphocytes d'un vertébré non humain immunisé au préalable avec la laminine P1 et qui a été sélectionné ensuite sur base du fait que l'anticorps formé présente, outre une bonne propriété de liaison avec la laminine humaine purifiée du placenta, également une bonne réaction avec la forme hautement moléculaire de la laminine produite à partir de sérum humain.

3. Anticorps monoclonal selon la revendication 1 ou 2, **caractérisé par** l'aptitude à se lier par paire avec un autre anticorps selon la revendication 1 ou 2 à l'antigène.

4. Anticorps monoclonal selon l'une quelconque des revendications 1 à 3, **caractérisé en ce qu'**il est attribué à la sous-classe IgG 2a.

5. Anticorps monoclonal selon l'une quelconque des revendications 1 à 3, **caractérisé en ce qu'**il est attribué à la sous-classe IgG 1.

6. Lignée cellulaire d'hybridome, qui produit un anticorps selon l'une quelconque des revendications 1 à 3, pouvant être produite par fusion de cellules d'une lignée cellulaire de myélome et de lymphocytes d'un vertébré non humain immunisé au préalable avec la laminine P1 et sélection consécutive des hybrides sur base du fait que l'anticorps produit par l'hybride présente, outre une bonne propriété de liaison avec la laminine humaine purifiée du placenta, également une bonne réaction avec la forme hautement moléculaire de la laminine produite à partir de sérum humain.

7. Lignée cellulaire d'hybridome selon la revendication 6, **caractérisée en ce que** les lymphocytes sont prélevés de souris immunisées à la laminine P1 de la souche Balb/c et la lignée cellulaire de myélome est la lignée cellulaire de myélome de souris P3X63AG8.653.

8. Lignée cellulaire d'hybridome DSM ACC2181.

9. Lignée cellulaire d'hybridome DSM ACC2180.

10. Lignée cellulaire d'hybridome DSM ACC2182.

11. Procédé pour la production d'un anticorps monoclonal selon l'une quelconque des revendications 1 à 5, **caractérisé en ce que**
a) des vertébrés non humains sont immunisés par le fragment de laminine P1 produit par la digestion par des pepsines de placenta humain,
b) des lymphocytes sont extraits à partir des vertébrés immunisés et fusionnés avec les cellules du myélome,
c) les hybrides sont choisis en fonction de la présence d'un anticorps présentant les propriétés indiquées dans les revendications 1 à 3 et sont clonés et
d) l'anticorps est extrait à partir de ces clones.

12. Procédé selon la revendication 11, **caractérisé en ce que** pour la réalisation de l'étape de procédé d) dans la revendication 11, on utilise les lignées cellulaires d'hybridome DSM ACC2181, DSM ACC2180 ou DSM ACC2182.

13. Procédé pour la détermination immunologique de laminine naturelle en utilisant un anticorps en couche fixé par adhérence ou covalence à un matériau support et un anticorps secondaire marqué qui détecte l'antigène lié à l'anticorps en couche, **caractérisé en ce que** l'anticorps en couche est un anticorps monoclonal selon l'une quelconque des revendications 1 à 5.

14. Procédé pour la détermination immunologique de laminine naturelle en utilisant un anticorps en couche fixé par adhérence ou covalence à un matériau support et un anticorps secondaire marqué qui détecte l'antigène lié à l'anticorps en couche, **caractérisé en ce que** l'anticorps secondaire marqué est un anticorps monoclonal selon l'une quelconque des revendications 1 à 5.

15. Procédé selon la revendication 14, **caractérisé en ce que** l'anticorps en couche est un anticorps monoclonal selon l'une quelconque des revendications 1 à 5.

16. Procédé selon les revendications 14 ou 15, **caractérisé en ce que** l'anticorps en couche est un anticorps monoclonal qui est produit par la lignée cellulaire d'hybridome DSM ACC2181.
